(19) Europäisches Patentamt
European Patent Office
Office européen des brevets

(11) EP 0 810 204 B1

(12) **EUROPÄISCHE PATENTSCHRIFT**

(45) Veröffentlichungstag und Bekanntmachung des Hinweises auf die Patenterteilung:
**05.01.2000 Patentblatt 2000/01**

(51) Int Cl.[7]: **C07C 251/24**, C08G 73/02

(21) Anmeldenummer: **97108003.1**

(22) Anmeldetag: **16.05.1997**

(54) **Dendritische stickstoffhaltige organische Verbindungen mit planar-chiralen oder axial-chiralen Endgruppen, ihre Herstellung und Verwendung**

Dendritic organic nitrogen-containing compounds with planar-chiral or axial terminal-groups, their preparation and use

Composés dendritiques organiques contenant de l'azote avec des groupes terminaux chiraux-plans ou chiraux-arials, leur préparation et utilisation

(84) Benannte Vertragsstaaten:
**AT BE CH DE DK ES FR GB IE IT LI NL PT SE**

(30) Priorität: **29.05.1996 DE 19621510**

(43) Veröffentlichungstag der Anmeldung:
**03.12.1997 Patentblatt 1997/49**

(73) Patentinhaber: **BASF AKTIENGESELLSCHAFT**
**67056 Ludwigshafen (DE)**

(72) Erfinder:
- **Paulus, Wolfgang Dr.**
  **55128 Mainz (DE)**
- **Vögtle, Fritz Prof. Dr.**
  **53347 Alfter Impekoven (DE)**
- **Issberner, Jörg**
  **53913 Swisttal-Movenkoven (DE)**

(56) Entgegenhaltungen:
**WO-A-93/14147** **WO-A-93/19787**
**US-A- 4 631 337**

- **TETRAHEDRON: ASYMMETRY , Bd. 7, Nr. 8, Juli 1996, Seiten 2223-2232, XP002037380 JÖRG ISSBERNER ET AL.: "Poly(amine/imine) dendrimers bearing planar chiral terminal groups - ..."**
- **CHEMISCHE BERICHTE, Bd. 126, 1993, WEINHEIM DE, Seiten 2133-2135, XP002037381 ROLF MOORS ET AL.: "Dendrimere polyamine"**
- **BULLETIN OF THE CHEMICAL SOCIETY OF JAPAN, Bd. 58, Nr. 12, 1985, TOKYO JP, Seiten 3621-3622, XP002037382 NAOHIDE MATSUMOTO ET AL.: "Synthesis and characterization of a chloro-bridged binuclear iron (III) complex"**

EP 0 810 204 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue dendritische stickstoffhaltige organische Verbindungen, die mindestens 4 planar-chirale oder axial-chirale Gruppen auf ihrer Oberfläche tragen, deren Herstellung ausgehend von achiralen Polyamin (imin)dendrimeren sowie deren Verwendung.

Seit der Veröffentlichung von E. Buhleier, W. Wehner und F. Vögtle in Synthesis (1978), 137-139 zur generellen Synthese von strukturperfekten und -imperfekten hochverzweigten Polyaminen hat man sich auch mit der großtechnischen Synthesemöglichkeit von Dendrimeren beschäftigt (vgl. z.B. WO 93/14147; E.M.M. de Brabander-van den Berg, E.W. Meijer, Angew. Chem. (1993), 105, 1370-1372 bzw. Angew. Chem. Int. Ed. (1993) 31, 1308-1311; C. Wörner, R. Mühlhaupt, Angew. Chem. (1993), 105, 1367-1370; Angew. Chem. Int. Ed. Engl. (1993), 32, 1306-1308; R. Moors, F. Vögtle, Chem. Ber. (1993) 126, 2133-2135). Die Verfügbarkeit von Dendrimeren in größeren Mengen ist den Publikationen von J. Issberner, R. Moors und F. Vögtle, Angew. Chem. (1994), 106, 2507-2514, bzw. Angew. Chem. Int. Ed. (1994), 33, 2313-2320; H.B. Mekelburger, W. Jaworek, F. Vögtle, Angew. Chem. (1992) 104, 1609-1614, bzw. Angew. Chem. Int. Ed. (1992) 31, 1571-1576; D.A. Tomalia, H.D. Durst, Top. Curr. Chem. (1993), 165, 193-313 zu entnehmen. In den Publikationen von J.F.G.A. Jansen, H.W.I. Peerlings, E.M.M. de Brabander-van den Berg, E.W. Meijer, Angew. Chem (1995), 107, 1321-1324, bzw. Angew. Chem. Int. Ed. (1995), 34, 1206-1209 wird die Synthese von Polyamindendrimeren mit chiralen Endgruppen beschrieben. Allerdings zeigen die Dendrimere mit nur einem stereogenen Zentrum je funktioneller Gruppe eine abnehmende optische Aktivität mit steigender Generationszahl, während Dendrimere mit rigiden chiralen Gruppen eine starke optische Aktivität aufweisen, die mit steigender Generationszahl nicht abnimmt.

Dendrimere, die an ihrer äußeren Hülle katalytisch aktive Gruppen tragen, sollten einige Probleme vermeiden, die übliche Katalysatoren aufweisen. Insbesondere hofft man, daß die kurze Reaktionszeit und hohe Aktivität homogen gelöster Katalysatoren mit der leichten Aufarbeitung von Reaktionsmischungen mit heterogenen Katalysatoren kombiniert werden kann (vgl. J.W.J. Knapen, A.W. van der Made, J.C. de Wilde, P.W.M.N. van Leeuwem, P. Wijkens, D. M. Grove, G. van Koten, Nature (1994), Vol. 372, 659-663; Du Bois et al. Inorg. Chem. (1994), 33, 5482; B.B.De B.B. Lohray, S. Sivaram, P.K. Dhal, Tetrah. Asymmetry (1995), 6 (9), 2105-2108 und darin enthaltene Literaturangaben; G. Henrici-Olivé, S. Olivé, Angew. Chem. (1974) 86, 1-56). Katalytisch aktive Dendrimere besitzen in der Regel eine gute Löslichkeit, so daß homogene Reaktionen möglich sind, und sie können leicht aus der Reaktionsmischung entfernt und recycled werden (z.B. mittels Osmose oder Filtration).

Eine Reihe wichtiger chiraler Substanzen brauchen zu ihrer Synthese enantiomerreine Darstellungsweisen, die durch den Einsatz von chiralen Hilfsstoffen (Katalysatoren) erreicht werden. Besonders vielseitige Verwendung haben dabei Metall-Salen und Metall-Salcomin-Komplexe gefunden, z.B. für asymmetrische Diels-Alder-Reaktionen (vgl. T. Katsuki et al., Synlett, (1995), 829-830), olefinische β-Ketoester (vgl. J.D. White, S.C. Jeffrey, Synlett, (1995), 831 ff.), Epoxidierung von Z-Olefinen , z.B. Cycloalkene, wie Stilben, Cyclopenten, -hexen, -hepten, -octen, Dihydronaphthen, Inden (vgl. S. Chang, J.M. Gavlin, E.N. Jacobsen, J. Am. Chem. Soc. (1994) 116, 6937-6938; P.A. Ganeshpure, S. Satish, Tetrahedr. Lett (1988) 29, 6629-6632), Di- und Trisubstituierte Olefine, wie Zimtsäureester, Maleinsäure, Maleinsäure-ester, -imide, -amide, -anhydrid, -halbester, -halbamide, β-substituierte Acrylsäure, β-substituierte Acrylsäure-ester, -amide, in β-Position substituiertes Styrol bzw. kernstubstituierte Styrole und Dienen (z.B. 1,3-Cycloocten, Butadien) (vgl. S. Chang, R.M. Heid, E.N. Jacobsen, Tetrahedron Lett. Vol. 35 (1994) 662-672), cis-Alkenalkine (cgl. T. Hamada, R. Irie, T. Katsuki, Synlett. (1994), 479-481) und Cyclopropanierung (vgl. T. Fukuda, T. Katsuki, Synlett. (1995), 825-828). Es wurden sehr gute und bisher unerreichte Enantiomerenüberschüsse der Produkte erzielt. Bei bestimmten Reaktionen ist die Chiralität der Salen-Komplexe zwar nicht gefordert, stört aber die Reaktion auch nicht, z.B. bei der Hydrierung von terminalen Alkenen und Alkinen (vgl. J. Chem. Soc., Perk. Trans. (1990), 887 ff.), bei der Fixierung von Sauerstoff (vgl. R.S. Drago, J. Gaul, A. Zombeck, D.K. Straub, J. Am. Chem. Soc. (1990) 102, 1033-1038; R.S. Drago, J.P. Cannady, K.A. Leslie, J. Am. Chem. Soc. (1980) 102, 6014-6019) und der Wasserspaltung (vgl. M. Watkinson, A. Whiting, C.A. Mc Auliffe, J. Che, Soc. Chem. Commun. (1994), 2141-2143).

Aufgabe der vorliegenden Erfindung ist es, neue dendritische stickstoffhaltige organische Verbindungen mit planar-chiralen bzw. axial-chiralen Gruppen aufzuzeigen, die technisch gut zugänglich sind, keine Gefahr einer Racemisierung in sich bergen und für weitere Reaktionen als katalytische Zentren wirken.

Gegenstand der vorliegenden Erfindung sind dendritische stickstoffhaltige organische Verbindungen, die mindestens 4 planar-chirale oder axial-chirale bzw. helical-chirale Gruppen (Axial-chirale und planar-chirale Verbindungen und Moleküle können hinsichtlich ihrer molekularen Chrialität nicht nur als axial S und R (aS, aR) bzw. planar-chiral S und planar-chiral R (pS, pR) spezifiziert werden, sondern, wie in dem Buch von Hauptmann/Mann (S. Hauptmann, G. Mann, Stereochemie, Spektrum Akademischer Verlag, Heidelberg, 1996, 58-67) und in dem Beitrag von G. Helmchen (G. Helmchen in Houben-Weyl, Methods of Organic Chemistry, Additional and Supp. Vol. of the 4th Edition, Stereoselective Synthesis (Editors: G. Helmchen, R. W. Hoffmann, J. Mulzer, E. Schaumann, Georg Thieme Verlag, Stuttgart, 1995, Bd. E 21a, Kap. 1, S. 1-33) oft vorteilhaft auch als helical-chirale Spezies betrachtet werden, also als (-) und (+) P und hinsichtlich ihres Chiralitätssinns charakterisiert und spezifiziert werden.) enthalten, wobei diese planar-chiralen

oder axial-chiralen Gruppen als Schiff'sche Basen mit den primären Aminogruppen von Verbindungen der allgemeinen Formel (I)

$$(R^1R^1)N\text{-}X\text{-}N(R^1R^1) \qquad (I)$$

in der

$R^1$ $(R^2R^2)N\text{-}(CH_2)_2$- oder $(R^2R^2)N\text{-}(CH_2)_3$-,

$R^2$ Wasserstoff oder $(R^3R^3)N\text{-}(CH_2)_2$- oder $(R^3R^3)N\text{-}(CH_2)_3$-,

$R^3$ Wasserstoff oder $(R^4R^4)N\text{-}(CH_2)_2$- oder $(R^4R^4)N\text{-}(CH_2)_3$-,

$R^4$ Wasserstoff oder $(R^5R^5)\text{-}N\text{-}(CH_2)_2$- oder $(R^5R^5)N\text{-}(CH_2)_3$-,

$R^5$ Wasserstoff oder $(R^6R^6)N\text{-}(CH_2)_2$- oder $(R^6R^6)N\text{-}(CH_2)_3$- und

$R^6$ Wasserstoff bedeuten,

X für

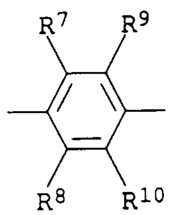
,
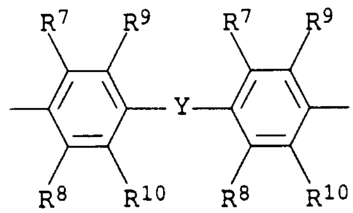
,
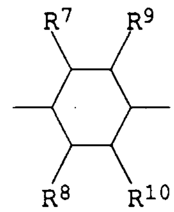
,

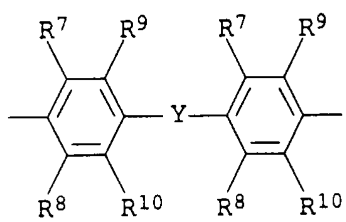
,

$-(CH_2)_n-$, $-(CH_2)_3\text{-}NR^{11}\text{-}(CH_2)_3-$, $(CH_2)_2\text{-}NR^{11}\text{-}(CH_2)_2-$, $C_2$- bis $C_{20}$-Alkylen
$-(CH_2)_1$- [O- $(CH_2)_k]_m$-O-$(CH_2)_1)$-

und Y für $CR^7R^9$, Sauerstoff, C=O, $SO_2$, n für 2 bis 20, 1 und k für 2 bis 6 und m für 1 bis 40,

$R^7$,$R^8$, $R^9$, $R^{10}$ für Wasserstoff oder $C_1$- bis $C_6$-Alkyl,

$R^{11}$ für $C_1$- bis $C_{20}$-Alkyl, $C_2$- bis $C_{20}$-Dialkylamino-$C_2$-bis $C_{10}$-alkyl, $C_1$- bis $C_{10}$-Alkoxy-$C_2$- bis $C_{10}$-alkyl, $C_2$- bis $C_{20}$-Hydroxyalkyl, $C_3$- bis $C_{12}$-Cycloalkyl, $C_4$- bis $C_{20}$-Cycloalkyl-alkyl, $C_2$- bis $C_{20}$-Alkenyl, $C_4$- bis $C_{30}$-Dialkylamino-alkenyl, $C_3$- bis $C_{30}$-Alkoxyalkenyl, $C_3$- bis $C_{20}$-Hydroxyalkenyl, $C_5$- bis $C_{20}$-Cycloalkyl-alkenyl, gegebenenfalls durch $C_1$- bis $C_8$-Alkyl, $C_2$- bis $C_8$-Dialkylamino-, $C_1$- bis $C_8$-Alkoxy, Hydroxy, $C_3$- bis $C_8$-Cycloalkyl, $C_4$- bis $C_{12}$-Cycloalkyl-alkyl, ein- bis fünffach substituiertes Aryl oder $C_7$- bis $C_{20}$-Aralkyl oder gemeinsam eine gegebenenfalls durch Stickstoff oder Sauerstoff unterbrochene Alkylenkette wie Ethylenoxid, Propylenoxid, Butylenoxid und $-CH_2\text{-}CH(CH_3)\text{-}O$- oder

Polyisobutylen mit 1 bis 100 iso-Butyleneinheiten stehen,

verknüpft sind.

Erfindungsgemäß dendritische Verbindungen mit planar-chiralen Gruppen sind insbesondere solche, die als planar-chirale Gruppen solche der Formeln

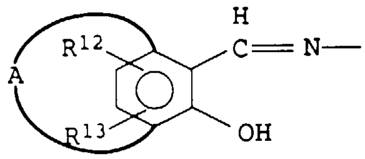

mit dendritischen stickstoffhaltigen organischen Verbindungen der allgemeinen Formel (I) verknüpft sind, worin =N- jeweils der Stickstoff der aus der primären Aminogruppe erhaltenen Schiff'schen Base ist,

$R^{12}$ und $R^{13}$ unabhängig voneinander folgende Bedeutung haben können:
H, Alkyl mit 1 bis 22 Kohlenstoffatomen,
O-$C_1$- bis $C_{22}$-Alkyl,
S-$C_1$- bis $C_{22}$-Alkyl,

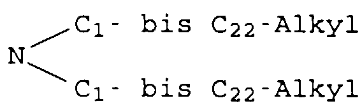 , 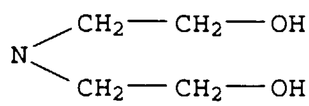

$$O-(CH_2-CH_2-O)_{1-6}CH_2-CH_2-R^{14}$$

$$O-(CH(CH_3)-CH_2-O)_{1-6}CH_2-CH_2-R^{14}$$

$$S-(CH_2-CH_2-O)_{1-6}CH_2-CH_2-R^{14}$$

$$S-(CH(CH_3)-CH_2-O)_{1-6}CH_2-CH_2-R^{14},$$

wobei

$R^{14}$ für H, OH, $OCH_3$, $OC_2H_5$,

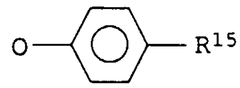

oder

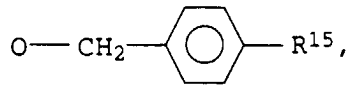

und

$R^{15}$ für H, Alkyl mit 1 bis 4 Kohlenstoffatomen, O-$C_1$- bis $C_4$-Alkyl, Halogen, CN oder $NO_2$ stehen können,

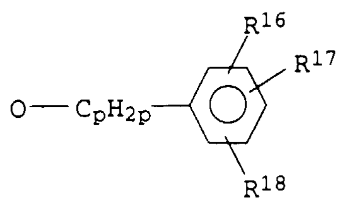
,

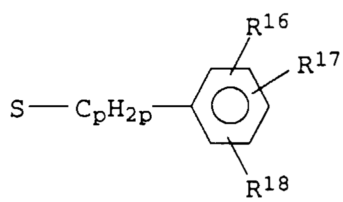
,

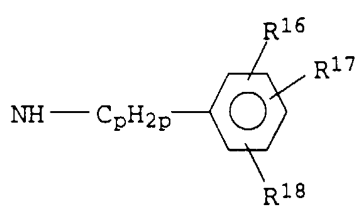
,

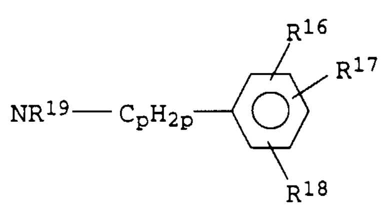
,

worin p = O oder 1 bis 22 sein kann und $R^{16}$, $R^{17}$ und $R^{18}$ unabhängig voneinander für H, Alkyl mit 1 bis 22 Kohlenstoffatomen, O-$C_1$- bis $C_{22}$-Alkyl,
S-$C_1$- bis $C_{22}$-Alkyl;

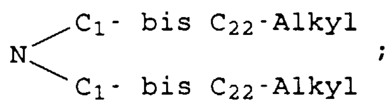

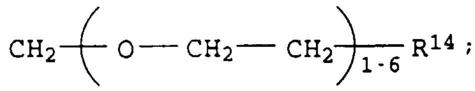

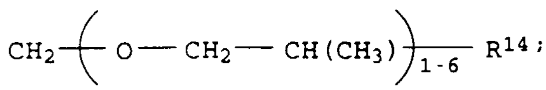

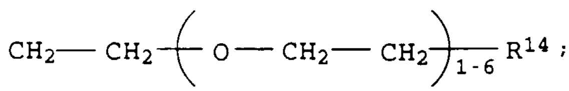

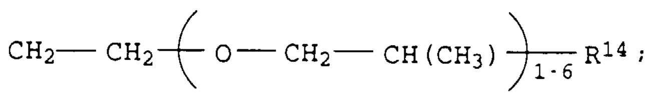

Halogen, $NO_2$, CN,

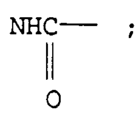

$R^{19}$ für Alkyl mit 1 bis 20 Kohlenstoffatomen,

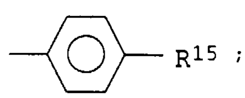

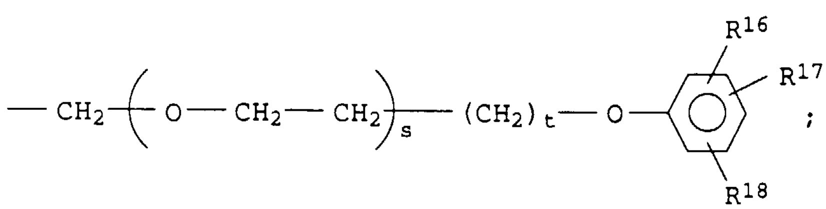

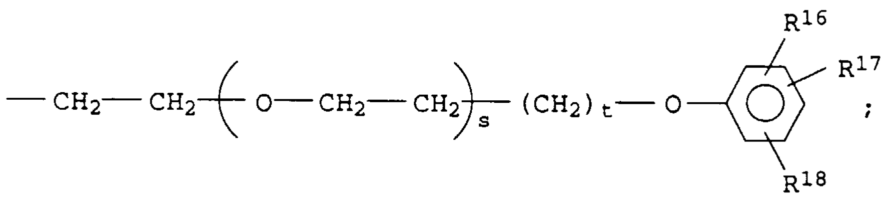

mit s = 0,1 oder 2
und t = 0 oder 1 stehen,
ein fünf- oder sechsgliedriger stickstoff-, sauerstoff- oder schwefelhaltiger heteroaromatischer Rest, der gegebenenfalls durch Alkyl mit 1 bis 22 Kohlenstoffatomen, O-$C_1$- bis $C_{22}$-Alkyl, S-$C_1$- bis $C_{22}$-Alkyl, NH-$C_1$- bis $C_{22}$-Alkyl oder

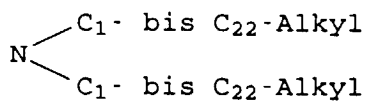

substituiert sein kann,
$-C{\equiv}CH$ $-CH_2-C{\equiv}CH$, $-CH_2-C{\equiv}C-CH_3$, $-CH_2-C{\equiv}C-C_2H_5$, $-CH_2-CH_2-C{\equiv}CH$,

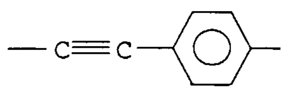

$C_1$- bis $C_{22}$-Alkyl ;

A für $-(CH_2)-_{4\ bis\ 8}$, $-CH_2-O-CH_2-CH_2-OCH_2-$,
$-CH_2-O-CH_2-CH\ (CH_3)-O-CH_2-$,
$-CH_2-O-CH_2-CH_2-CH_2-O-CH_2-$,
$-CH_2-O-(CH_2)_4-O-CH_2-$, $-O\{CH_2-CH_2-O\}_1$ oder 2,
$-O\{CH_2-CH(CH_3)-O\}_{1\ oder\ 2}$ oder

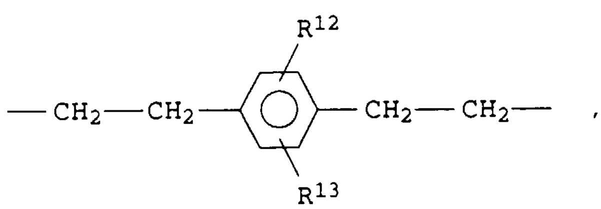

worin

$R^{12}$ und $R^{13}$ unabhängig voneinander die oben angegebene Bedeutung haben sowie zusätzlich Halogen, CN, $NO_2$. $-N{=}N-R^{20}$ oder

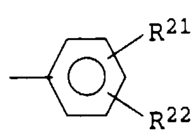

bedeuten können, wobei

$R^{20}$ für eine Phenylgruppe oder eine mit $NO_2$, CN, Halogen, $C_1$- bis $C_4$-Alkyl, $C_1$, bis $C_4$-

Alkoxy, $C_1$- bis $C_4$-alkylthio oder $C_1$- bis $C_4$-alkylamino substituierte Phenylgruppe,

$R^{21}$ und $R^{22}$ unabhängig voneinander für $C_1$- bis $C_4$-Alkyl, $C_1$- bis $C_4$-Alkoxy, CN, $NO_2$ oder Halogen stehen,

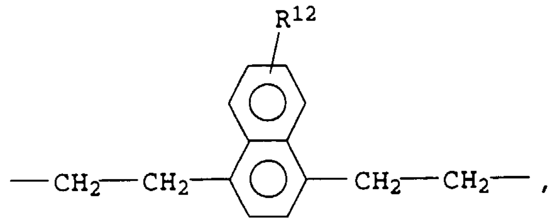

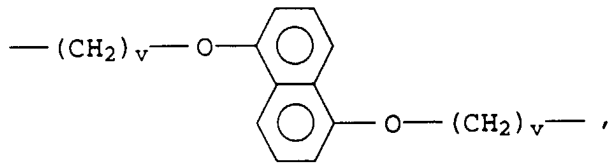

mit v = 1, 2 oder 3
oder

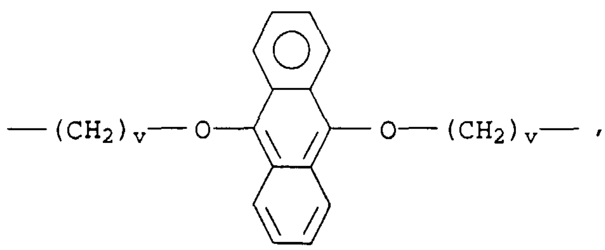

stehen.

Erfindungsgemäße dendritische Verbindungen mit axial-chiralen Gruppen sind insbesondere solche, die als axial-chirale Gruppen solche der Formeln

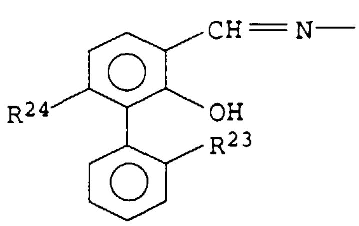

oder

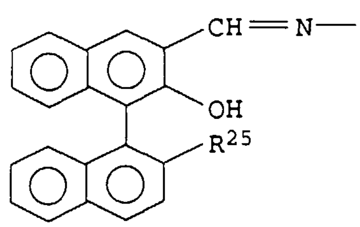

worin

$R^{23}$ und $R^{24}$ unabhängig voneinander für $C_1$- bis $C_{22}$-Alkyl, $C_1$- bis $C_6$-Alkyloxy, Phenyl, mit Halogen, CN, $NO_2$, $C_1$- bis $C_4$-Alkyl, $C_1$- bis $C_4$-Alkoxy substituiertes Phenyl, oder

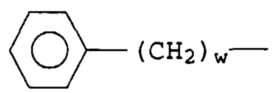

mit w = 1 bis 4 stehen,

mit der Maßgabe, daß mindestens einer der Reste $R^{23}$ und $R^{24}$ eine tert.-Butylgruppe oder ein anderer die freie Drehbarkeit der Phenylreste verhindernder Rest ist und $R^{25}$ ein tert. Butylrest, ein Phenylrest oder ein mit Halogen, CN, $NO_2$, $C_1$- bis $C_4$-Alkyl oder $C_1$ bis $C_4$-Alkoxy substituierter Phenylrest ist,

mit dendritischen stickstoffhaltigen organischen Verbindungen der allgemeinen Formel (I) verknüpft sind, worin =N- jeweils der Stickstoff der aus der primären Aminogruppe erhaltenen Schiff'schen Base ist.

Ebenso sind die als Metallkomplexe von Übergangsmetallen, wie Ag(I)-, Mn(II)-, Mn(III)-, Mn(IV)-, Co(II)-, Co (III)-, Ni(II)-, Cu(II)-, Pd(II)-salzen, vorliegenden erfindungsgemäßen dendritischen Verbindungen mit planar-chiralen bzw. axial-chiralen Gruppen Gegenstand der vorliegenden Erfindung.

Zur Herstellung dieser Metallkomplexe können die erfindungsgemäßen dendritischen planar-chirale bzw. axial-chirale Gruppen enthaltenden Verbindungen in einem geeigneten Lösungsmittel zusammen mit Übergangsmetallsalzen bei Raumtemperatur bis Siedetemperatur des Lösungsmittels gelöst oder dispergiert, das Lösungsmittel anschließend entfernt und die erhaltenen Komplexe zur Entfernung von überschüssigem Metallsalz mit Wasser ausgeschüttelt werden.

Die Übergangsmetallkomplexe können aber auch simultan bei der Herstellung der Schiff'schen Basen durch Zufügen der Übergangsmetallsalze zur Reaktionsmischung erhalten werden.

Gegenstand der vorliegenden Erfindung ist auch ein Verfahren zur Herstellung erfindungsgemäßer dendritischer stickstoffhaltiger organischer Verbindungen, die mindestens 4 planar-chirale Gruppen enthalten, wobei Verbindungen der allgemeinen Formel (I) mit Hydroxyaldehyden der Formel

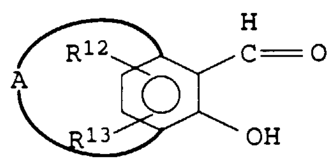

in Gegenwart von wasserbindenden oder bei der Destillation wasserschleppenden Substanzen miteinander umgesetzt werden.

Ebenso ist Gegenstand der vorliegenden Erfindung ein Verfahren zur Herstellung erfindungsgemäßer dendritischer stickstoffhaltiger organischer Verbindungen, die mindestens 4 axial-chirale Gruppen enthalten, wobei Verbindungen der allgemeinen Formel (I) mit Hydroxyaldehyden der Formeln

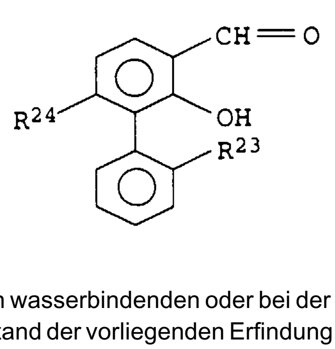

oder

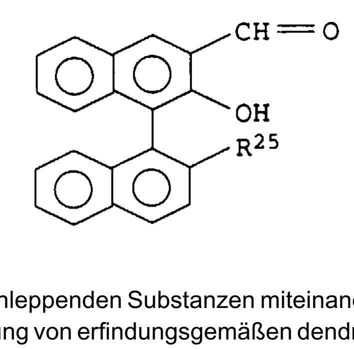

in Gegenwart von wasserbindenden oder bei der Destillation wasserschleppenden Substanzen miteinander umgesetzt werden. Gegenstand der vorliegenden Erfindung ist auch die Verwendung von erfindungsgemäßen dendritischen stickstoffhaltigen organischen Verbindungen mit planar-chiralen bzw. axial-chiralen Gruppen und deren Übergangsmetallkomplexen als Katalysatoren für die asymmetrische homogene Katalyse.

Zu den erfindungsgemäßen Verbindungen und Herstellverfahren ist im einzelnen noch folgendes auszuführen.

Soweit die Reste $R^1$ bis $R^{25}$ Alkylgruppen bedeuten oder enthalten und mehr als 3 Kohlenstoffatome enthalten, können diese geradkettig oder verzweigt sein oder auch cyclische, z.B. drei-, fünf- oder sechsgliedrige Reste enthalten.

Aminoverbindungen der allgemeinen Formel (I) sowie deren Herstellung können der DE-A 44 40 551 entnommen werden.

Die Aminoverbindungen der allgemeinen Formel (I) werden erfindungsgemäß mit Hydroxyaldehyden der Formeln

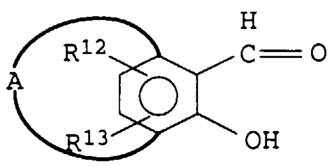 bzw. 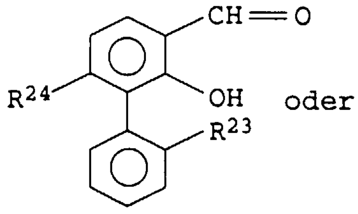 oder 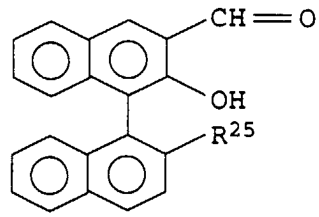

zu dendritischen Schiff'schen Basen mit planar-chiralen bzw. axial-chiralen Gruppen umgesetzt.

Ein Beispiel für eine planar-chirale Gruppe der Formel

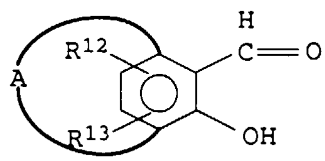

ist das 5-Formyl-4-hydroxy-[2,2]paracyclophan (= FHPCP) (1).

FHPCP (vgl. H. Hopf, D.G. Barrett, Liebigs Ann. 1995, 449-451) kann nach der Vorschrift von Belokon et al. (vgl. V. Rozenberg, V. Khartinov, D. Antonov, E. Sergeeva, A. Aleshkin, N. Ikonnikov, S. Orlova, Y. Belokon, Angew. Chem. (1994) 106, 106-108; Angew. Chem. Int. Ed. Engl. (1994) 33, 91; D.Y. Antonov, Y.N. Belokon, N.S. Ikonnikov, S.A. Orlova, A.P. Pisarevski, N.I. Raevski, V.I. Rozenburg, E.V. Sergeeva, Y.T. Struchkov, V.I. Tararov, E.V. Vorotsov, J. Chem. Soc., Perkin Trans. I., (1995) 1873-1879) und von Hopf und Barrett synthetisiert werden und in die Enantiomeren via Schiff'sche Base-Cu-Komplexe und mittels HPLC aufgetrennt werden. Der so erhaltene enantiomerenreine Aldehyd konnte für alle weitern Synthesen verwendet werden.

Die Kondensation des angereicherten und enantiomerenreinen Aldehyds FHPCP (1) mit dem dendritischen Polyamin der Formel (2a), (2b), (2c) (erste bis dritte Generation, umgesetzt mit vier (ergibt Verbindung (3a)), acht (ergibt Verbindung (3b)) oder sechzehn (ergibt Verbindung (3c)) $NH_2$-Gruppen kann bei Raumtemperatur in einem geeigneten organischen Lösungsmittel (z.B. Dichlormethan) unter Verwendung eines wasserentziehenden Mittels, wie z.B. Natriumsulfat, Mangnesiumsulfat, Molekularsieb, Calciumchlorid, NaOH, KOH, LiOH oder MgO oder durch Kochen unter Normaldruck bei Rückflußtemperatur in Benzol, Toluol oder Xylol, z.B. unter Verwendung einer Dean-Stark Falle, erfolgen. Man erhält dabei jeweils das Tetra-, Octa- bzw. Hexadecaimin (Verbindungen (3a) bis (3c)). Selbstverständlich kommen auch alle anderen bekannten und üblichen Kondensationsmethoden zur Herstellung von Schiff'schen Basen in Frage, sofern keine anderweitige Reaktion (z.B. mit der OH-Gruppe in ortho-Position des FHPCP) eintritt.

Nach der Kondensationsreaktion wird das erhaltene Produkt zur Entfernung überschüssigen FHPCPs zweckmäßigerweise mehrmals mit heißem Methanol gewaschen. Das dendritische Imin, dessen Reinheit über $^1$H-NMR-Spektroskopie festgestellt werden kann, bleibt dabei als orange-gelber Feststoff zurück.

Die erfindungsgemäßen Übergangsmetallkomplexe der dendritischen stickstoffhaltigen organischen Verbindungen mit planar-chiralen Gruppen können hergestellt werden, indem die chirale Gruppierungen enthaltenden dendritischen Verbindungen zusammen mit Übergangsmetallsalzen, wie z.B. Ag(I)-, Mn(II)-, Mn(III)-, Mn(IV)-, Co(II)-, Co (III)-, Ni(II)-, Pd(II)- oder Cu(II)-salze, beispielsweise Acetate, Triflate, Tosylate, Mesylate, Nitrate und - außer bei Ag (I) - Halogenide und Pseudohalogenide (wie z.B. $SCN^{\ominus}$) dieser Übergangsmetalle in entsprechenden organischen Lösungsmitteln, wie z.B. Methanol, Ethanol, Propanol, Butanol, Ethylenglykol, deren Methyl-, Ethyl-, Propyl- und höheren Ether, Dichlormethan, Chloroform, Benzol, Toluol, Xylol, Dimethylformamid, N-Methylpyrrolidon, Dimethylsulfoxid, Tetrahydrofuran, Tetrahydropyran, unter Stickstoffatmosphäre bei Raumtemperatur bis Siedetemperatur des entsprechenden organischen Lösungsmittels gelöst oder dispergiert wird, das Lösungsmittel anschließend entfernt und

die Übergangsmetallkomplexe zur Entfernung von überschüssigem Metallsalz mit Wasser ausgeschüttelt werden. Im Falle der Herstellung von Mn(III)-Komplexen können alternativ die chirale Gruppierungen enthaltenden dendritischen Verbindungen zusammen mit Mn(II)-salzen in organischem Lösungsmittel gelöst und während der Komplexierung Sauerstoff oder Luft durch die Lösung geblasen werden.

Alternativ können die Übergangsmetallionenkomplexe auch simultan bei der Herstellung der Schiff'schen Basen hergestellt werden, indem die oben angeführten Übergangsmetallsalze der Reaktionsmischung zugefügt werden. Der Vergleich des Circular-dichroismus (=CD)-Spektrums (Fig. 1) einer einzelnen chiralen Salen-Einheit (4) (Formel auf Seite 17) mit dem von chiralen multi Salen-Dendrimeren (3a) - (3c) zeigt keinen Unterschied, was darauf hinweist, daß die meisten oder alle Salen-Einheiten auf der Dendrimeroberfläche zur Komplexierung von Metallsalzen frei sind.

Der Vergleich der Circular-dichroismus (=CD)-Spektren von chirale Gruppierungen enthaltenen Dendrimeren ohne und mit komplexbildendem wasserfreiem Cobalt(II)chlorid ergibt einen deutlichen Unterschied. So zeigen die CD-Spektren der chiralen Dendrimere (3a) - (3c) mit und ohne die Salen-Einheiten komplexierendem Cobalt(II)chlorid einen deutlichen Unterschied, der darauf hinweist, daß die meisten oder alle Salen-Einheiten komplexieren und damit ein Dendrimer mit vielen katalytischen Zentren vorliegt.

In Schema 1 (Seiten 14 bis 16) sind die Reaktionen der 1. bis 3. Generation von Polyamindendrimeren gemäß Formel (2a), (2b), (2c) mit 4,8 bzw. 16 NH2-Gruppen mit FHPCP dargestellt, sowie Struktur (4) (Seite 17).

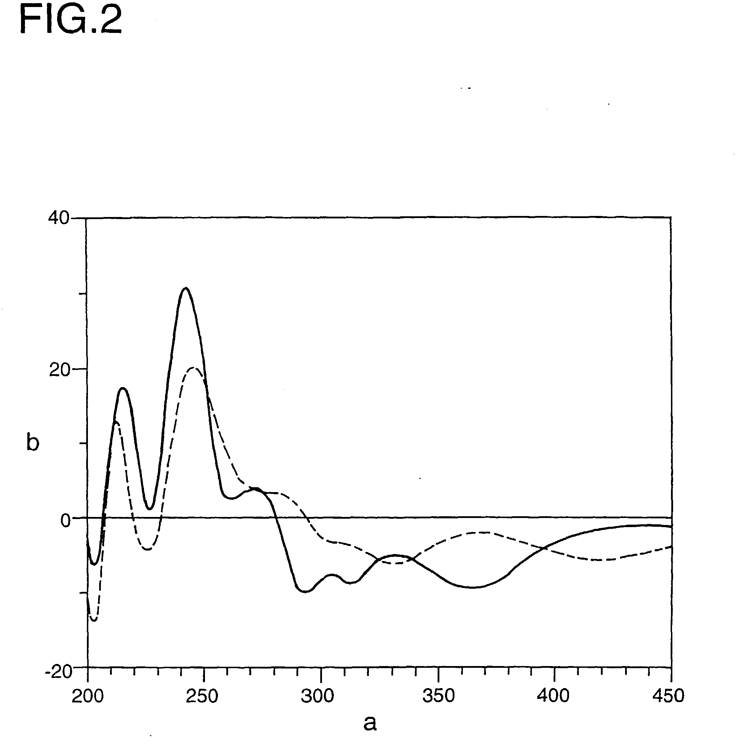

In Fig. 1 sind die CD-Spektren von (4) (...) und (3c) (_____ ) gelöst in Dichlormethan dargestellt; a = Wellenlänge [nm]; b = $\Delta\varepsilon$. In Fig. 2 sind die CD-Spektren von (3c) gelöst in Dichlormethan in Abwesenheit eines Übergangsmetallsalzes (_____) und nach Zugabe von wasserfreiem Cobalt(II)chlorid (...) gezeigt. a = Wellenlänge [nm] ; b = $\Delta\varepsilon$.

Schema 1:

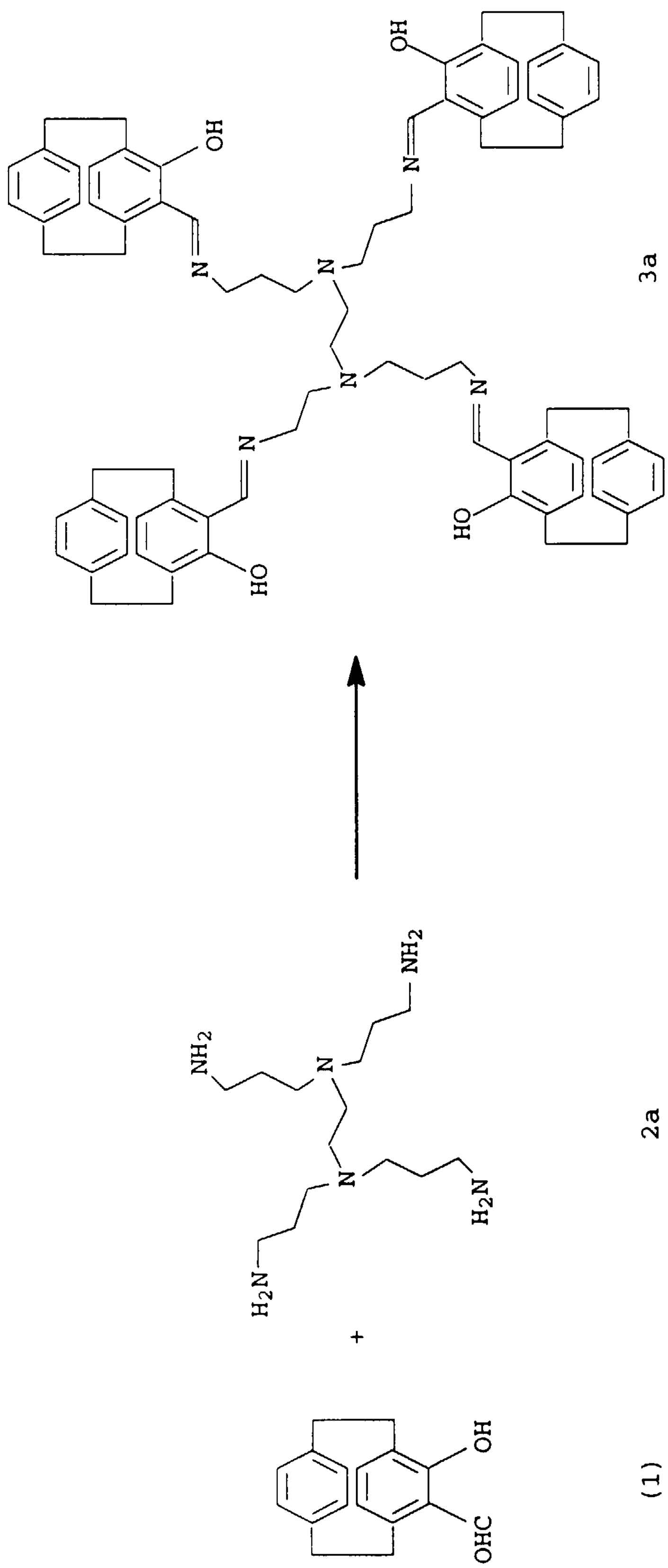

(1) + 2a → 3a

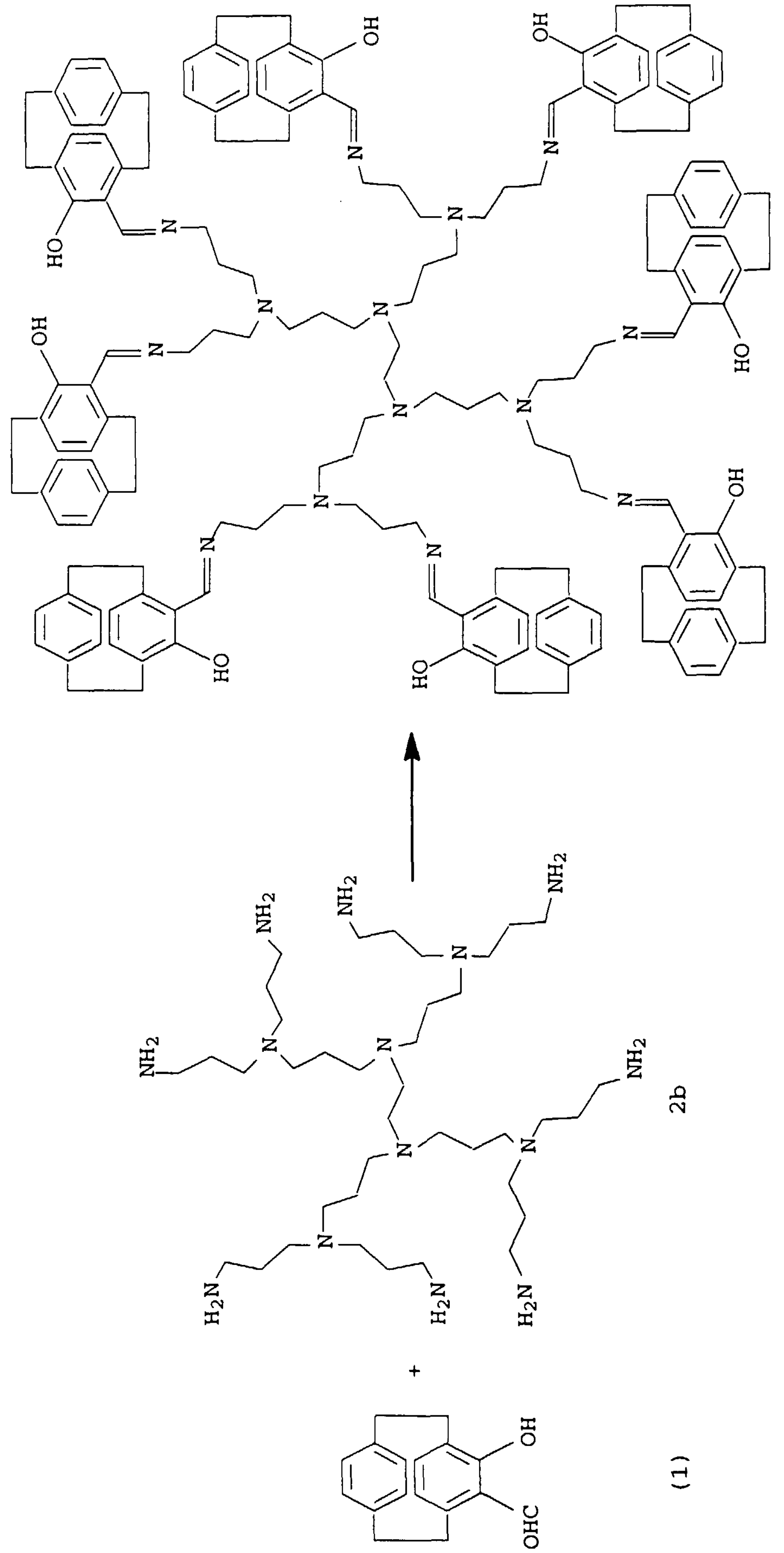

3b

2b

(1)

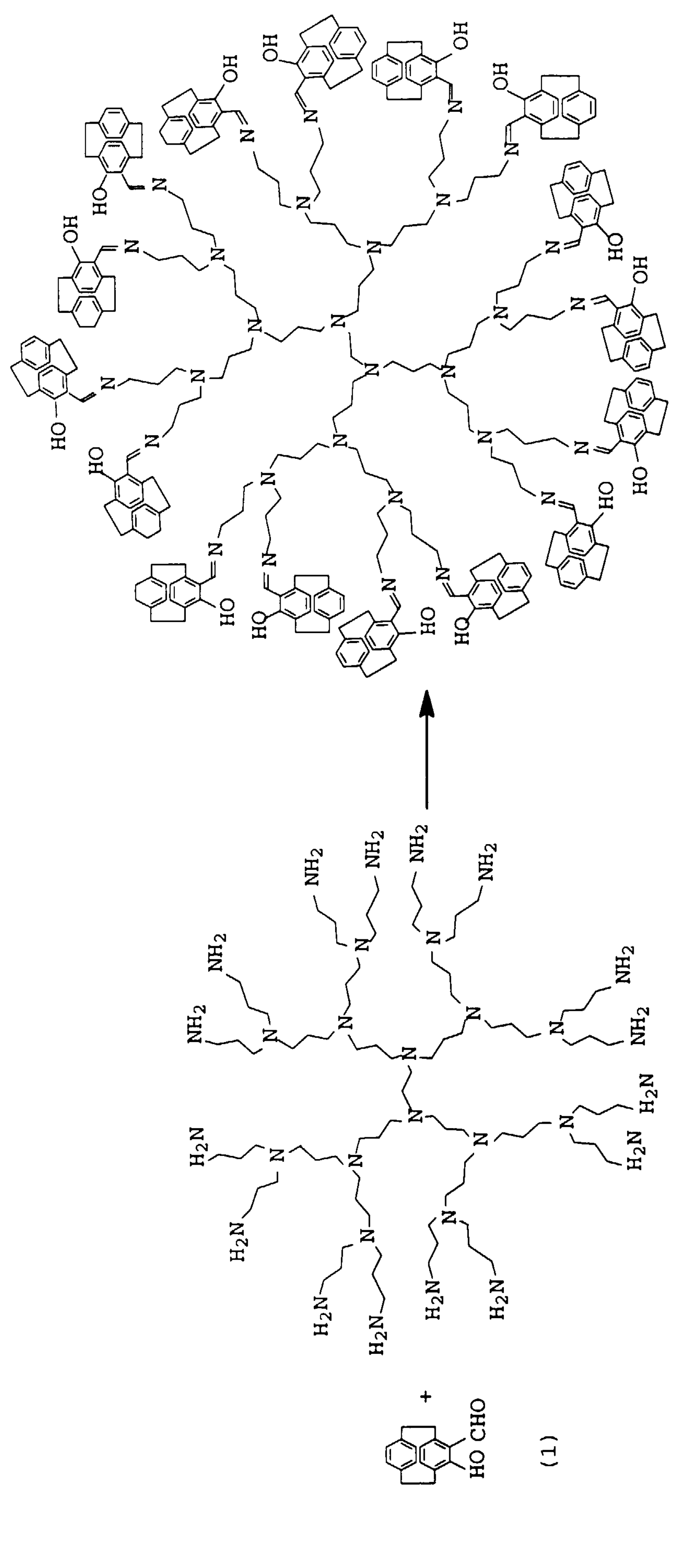

3c

2c

(1)

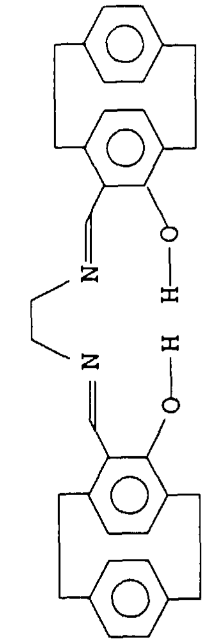

4

Beispiele

Generelle Vorgehensweise bei der Synthese der Schiff'schen Basen 3a, b, c, 4

7,5 **(3b)** mg (0,03 mmol) $(^{+})_D$-(R)-5-Formyl-4-hydroxy-[2,2]para-cyclophan (1) werden in 25 ml Lösungsmittel gelöst. 2,7 mg (0,004 mmol) des Octaamins (2b), gelöst in 10 ml Lösungsmittel, werden bei 60°C langsam hinzugetropft. Anschließend wird die Lösung 8 h an einem Schwerphasenabscheider zum Sieden erhitzt. Im Vakuum wird das Lösungsmittel entfernt und das resultierende orange-gelbe Öl mehrfach mit heißem Methanol gewaschen. Nach Entfernen aller flüchtigen Bestandteile im Feinvakuum bleibt ein orange-gelber Feststoff zurück.
Die Reinheit der Schiff'schen Base wurde mittels $^{1}$H-NMR-Spectroskopie bestimmt und beträgt 95 %. Ausbeute: 7,0 mg (69 %), orange-yellow, fp 42°, $[\alpha]_D^{25Grad}$=+478 (c = 0,2 in $CH_2Cl_2$); $^{1}$H-NMR (400 Hz, $CDCl_3$, 25°C) : δ = 1,5-1,6 (m, 16H, $CH_2$), 1,7-1,8 (m, 16H, $CH_2$), 2,42-2,57 (m, 36H, $CH_2$), 2,59-2,80 (m, 16H, $CH_2$), 2,88-2,97 (m, 8H, $CH_2$), 2,99-3,14 (m, 16H, $CH_2$), 3,24-3,32 (m, 8H, $CH_2$), 3,33-3,42 (m, 8H, $CH_2$), 3,46-3,57 (m, 16H, $CH_2$), 6,06 (d, $^{3}J$(H,H) = 7,6 Hz, 8H, CH), 6,12 (dd, $^{3}J$(H,H) = 7,6 Hz, $^{4}J$(H,H) = 1,7 Hz, 8H, CH), 6,34 (dd, $^{3}J$(H,H) = 7,6 Hz, $^{4}J$(H,H) = 1,7 Hz, 8H, CH), 6,39 (d, $^{3}J$(H,H) = 7,6 Hz, 8H, CH), 6,50 (dd, $^{3}J$(H,H) = 7,6 Hz, $^{4}J$(H,H) = 1,7 Hz, 8H, CH), 6,77 6. (dd, $^{3}J$(H,H) = 7,6 Hz, $^{4}J$(H,H) = 1,7 Hz, 8H, CH), 8,06 (s, 8H, CHN), 14,2 (br.s, OH); $^{13}$C-NMR (100 Hz, $CDCl_3$, 25°C) : δ = 24.7 ($C_s$, 28,9 ($C_s$) 32,2 ($C_2$) 33,9 ($C_s$), 35,3 ($C_s$), 51,7 ($C_s$), 52,2 ($C_s$), 56,9 ($C_s$), 119,1 ($C_q$), 123,7 ($C_t$), 126,6 ($C_t$), 128,1, 130,5 ($C_t$), 132,0 ($C_t$), 133,4 ($C_t$), 137,4 ($C_t$(, 137,5 $C_q$), 140,1 ($C_q$), 140,1 ($C_q$), 142,0 ($C_q$), 162,2 (CH=N), 163,0 ($C_q$); MS (positive-FABm NBA) *m*/*z* = 2620,7 (*M*$^{+}$[$C_{174}H_{204}N_{14}O_8$]+H, 20 %, $C_{174}H_{204}N_{14}O_8$ (2619,6); CHN-analysis (ber.:) H: 7,85 %, C: 79,87 %, N: 7,49 %, 0 4,89 %.

$^{1}$H-NMR **(3a)**(400 Hz, $CDCl_3$, 25°C) : δ = 1,81 (t, $^{3}J$(H,H) = 6,2 Hz; 8H, $CH_2$), 2,45-2,76 (m, 24H, $CH_2$), 2,93 (td, 4H, $CH_2$), 3,0-3,1 (m, 8H, $CH_2$), 3,25-3,40 (m, 8H, $CH_2$), 3,45-3,60 (m, 8H, $CH_2$), 6,07 (dd, $^{3}J$(H,H) = 7,6 Hz, $^{4}J$ (H,H) = 1 Hz, 4H, CH), 6,13 (d, $^{3}J$(H,H) = 7,9 Hz, 4H, CH), 6,34 (dd,$^{3}J$(H,H) = 7,9 Hz, $^{4}J$(H,H) = 1,7 Hz, 4H, CH), 6,40 (d, $^{3}J$(H,H) = 7,6 Hz, 4H, CJ), 6,50(d, $^{3}J$(H,H) = 7,9 Hz, 4H, CH), 6,78 6. (dd, $^{3}J$(H,H) = 7,7 Hz, $^{4}J$(H,H) = 1,7 Hz, 4H, CH), 8,06 (s, 4H, CHN), 14,2 (br.s, OH); $^{13}$C-NMR (100 Hz, $CDCl_3$, 25°C) : δ 24,7 ($C_s$), 28,9 ($C_s$), 29,9 ($C_s$), 32,2 ($C_s$), 33,9 ($C_s$), 35,3 ($C_s$), 51,7 ($C_s$), 52,2 ($C_s$), 56,9 ($C_s$), 119,1 ($C_q$), 123,7 ($C_t$), 126.6$_t$), 128,1; 130,5 ($C_t$), 1320 ($C_t$), 133,4 ($C_t$), 137,4 ($C_t$), 137,5 ($C_q$), 140,1 ($C_q$), 142,0 ($C_q$), 162,2 (CH=N), 163,0 ($C_q$),; MS (positive-FAB, m-NBA) m/z = 1225,8 (M$^{+}$ [$C_{82}H_{92}N_6O_4$]-H, 100 %), $C_{82}H_{92}N_6O_4$ (1224,72). CHN-analysis (ber.:) H: 7,57

%, C: 80,36 %, N: 6,86 %, O 5,22 %.

**(3c)** $^1$H-NMR (400 Hz, $CDCl_3$, 25°C) : δ = 1,5-1,85 (br.s, 64H, $CH_2$), 2,3-2,7 (m, 124H, $CH_2$), 2,85-2,95 (m, 16H, $CH_2$), 3,0-3,15 (m, 32H, $CH_2$), 3,2-3,4 (m, 32H, $CH_2$), 3,45-3,6 (m, 32H, $CH_2$); 6,06 (d, $^3J$(H,H) = 7,6 Hz, 16H, CH), 6,12 (d, $^3J$(H,H) = 7,6 Hz, 16H, CH), 6,34 (d, $^3J$(H,H) = 7,6 Hz, 16H, CH), 6,39 (d, $^3J$(H,H) = 7,6 Hz, 16H, CH), 6,50 (d, $^3J$(H,H) = 7,6 Hz, 16H, CH), 6,77 (d, $^3J$(H,H) = 7,6 Hz, 16H, CH), 8,06 (s, 16H, CHN), 14,2 (br.s, OH); $^{13}$C-NMR (100 Hz, $CDCl_3$, 25°C) : δ = 29,4 ($C_s$), 28,5 ($C_s$), 29,9 ($C_s$), 32,2 ($C_s$), 33,9 ($C_s$), 35,3 ($C_s$), 51,4 ($C_s$), 56,6 ($C_s$), 119,1 ($C_q$), 123,7 ($C_t$), 126,6 ($C_t$), 128,1, 130,5 ($C_t$), 132,0 ($C_t$), 133,4 ($C_t$), 147,4 ($C_t$), 137,5 ($C_q$), 140,1 ($C_q$), 142,0 ($C_q$), 162,5 (CH=N), 163,0 ($C_q$); $C_{358}H_{428}N_{30}O_{16}$ (5407,53); CHN-Analyse $C_{358}H_{428}N_{30}O_{16}$. 4 $HCCl_3$ ber. (gef.) %: H: 7,40 (7,71), C: 73,88 (73,77), N: 7,14 (7,06).

Enantiomeren-Trennung durch HPLC; Säule: Cellulose-tris(3,5-dimethylphenylcarbamat (CDMPC), 500 x 4,6 mm. Eluent: n-Hexan/Isopropanol 9:1, 0,3 mL $min^{-1}$. Druck: 3 bar. Temperatur 25°C. Detektion: UX, λ = 254 nm; $t_r$ [($^+$) $D^{-1}$] = 26 min; $t_r$ [($^-$)$D^{-1}$] = 35 min; k' [($^-$)$D^{-1}$] = 4,54; k' [($^+$)$D^{-1}$] = 5,92; α = 1,30; R = 1,89. $[\alpha]_{578}^{20}$ [(R)-1]=+588(c=0,02; $CH_2Cl_2$) Polarimeter Perkin-Elmer 241.

CD-Messungen mit JASCO-Spektropolarimeter J 720, 0,001 g/ml Lösung in Dichlormethan; 0,02 mm Zelle.

## Patentansprüche

1. Dendritische stickstoffhaltige organische Verbindungen, die mindestens 4 planar-chirale oder axial-chirale Gruppen enthalten, wobei diese planar-chiralen oder axial-chiralen Gruppen als Schiff'sche Basen mit den primären Aminogruppen von Verbindungen der allgemeinen Formel (I)

$$(R^1R^1)N\text{-}X\text{-}N(R^1R^1) \qquad (I)$$

in der

$R^1$ $(R^2R^2)N\text{-}(CH_2)_2\text{-}$ oder $(R^2R^2)N\text{-}(CH_2)_3\text{-}$,

$R^2$ Wasserstoff oder $(R^3R^3)N\text{-}(CH_2)_2\text{-}$ oder $(R^3R^3)N\text{-}(CH_2)_3\text{-}$,

$R^3$ Wasserstoff oder $(R^4R^4)N\text{-}(CH_2)_2\text{-}$ oder $(R^4R^4)N\text{-}(CH_2)_3\text{-}$,

$R^4$ Wasserstoff oder $(R^5R^5)\text{-}N\text{-}(CH_2)_2\text{-}$ oder $(R^5R^5)N\text{-}(CH_2)_3\text{-}$,

$R^5$ Wasserstoff oder $(R^6R^6)N\text{-}(CH_2)_2\text{-}$ oder $(R^6R^6)$ $N\text{-}(CH_2)_3\text{-}$

und

$R^6$ Wasserstoff bedeuten,

x für

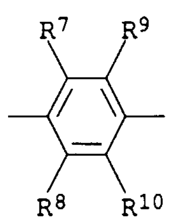 , 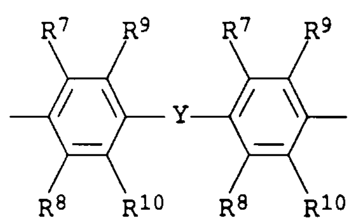 , 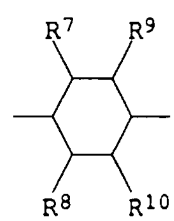 ,

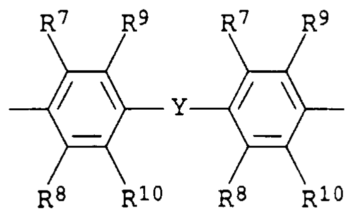 ,

$-(CH_2)_n-$, $-(CH_2)_3-NR^{11}-(CH_2)_3-$, $(CH_2)_2-NR^{11}-(CH_2)_2-$, $C_2$- bis $C_{20}$-Alkylen
$-(CH_2)_1-$ $[O-(CH_2)_k]_m-O-(CH_2)_1)-$

und Y für $CR^7R^9$, Sauerstoff, C=O, $SO_2$, n für 2 bis 20, l und k für 2 bis 6 und m für 1 bis 40,

$R^7$,$R^8$, $R^9$, $R^{10}$ für Wasserstoff oder $C_1$- bis $C_6$-Alkyl,

$R^{11}$ für $C_1$- bis $C_{20}$-Alkyl, $C_2$- bis $C_{20}$-Dialkylamino-$C_2$- bis $C_{10}$-alkyl, $C_1$- bis $C_{10}$-Alkoxy-$C_2$- bis $C_{10}$-alkyl, $C_2$- bis $C_{20}$-Hydroxyalkyl, $C_3$- bis $C_{12}$-Cycloalkyl, $C_4$- bis $C_{20}$-Cycloalkyl-alkyl, $C_2$- bis $C_{20}$-Alkenyl, $C_4$- bis $C_{30}$-Dialkylamino-alkenyl, $C_3$- bis $C_{30}$-Alkoxy-alkenyl, $C_3$- bis $C_{20}$-Hydroxyalkenyl, $C_5$- bis $C_{20}$-Cycloalkyl-alkenyl, gegebenenfalls durch $C_1$- bis $C_8$-Alkyl, $C_2$- bis $C_8$-Dialkylamino-, $C_1$- bis $C_8$-Alkoxy, Hydroxy, $C_3$- bis $C_8$-Cycloalkyl, $C_4$- bis $C_{12}$-Cycloalkyl-alkyl, ein- bis fünffach substituiertes Aryl oder $C_7$- bis $C_{20}$-Aralkyl oder gemeinsam eine gegebenenfalls durch Stickstoff oder Sauerstoff unterbrochene Alkylenkette wie Ethylenoxid, Propylenoxid, Butylenoxid und $-CH_2-CH(CH_3)-O-$ oder Polyisobutylen mit 1 bis 100 iso-Butyleneinheiten stehen,

verknüpft sind.

**2.** Verbindungen nach Anspruch 1, dadurch gekennzeichnet, daß als planar-chirale Gruppen solche der Formeln

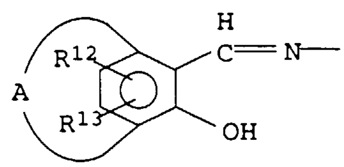

mit dendritischen stickstoffhaltigen organischen Verbindungen der allgemeinen Formel (I) verknüpft sind, worin =N- jeweils der Stickstoff der aus der primären Aminogruppe erhaltenen Schiff'schen Base ist,

$R^{12}$ und $R^{13}$ unabhängig voneinander folgende Bedeutung haben können:
H, Alkyl mit 1 bis 22 Kohlenstoffatomen,
O-$C_1$- bis $C_{22}$-Alkyl,
S-$C_1$- bis $C_{22}$-Alkyl,

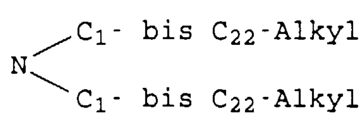 , 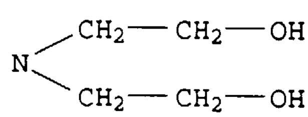

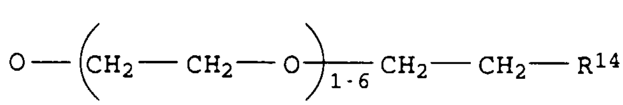

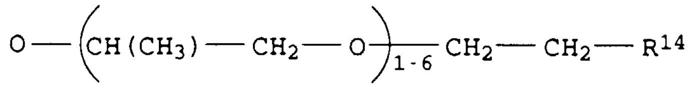

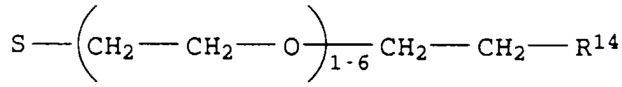

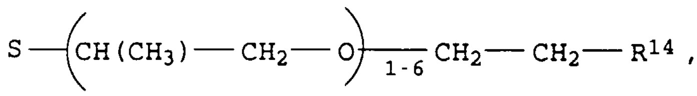

wobei

$R^{14}$ für H, OH, $OCH_3$, $OC_2H_5$,

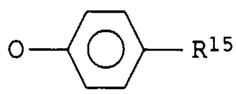

oder

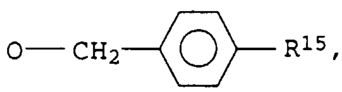

und

$R^{15}$ für H, Alkyl mit 1 bis 4 Kohlenstoffatomen, O-$C_1$- bis $C_4$-Alkyl, Halogen, CN oder $NO_2$ stehen können,

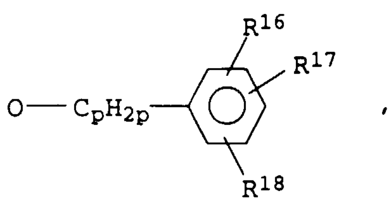

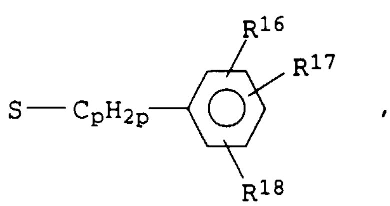

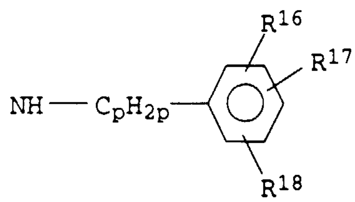 ,

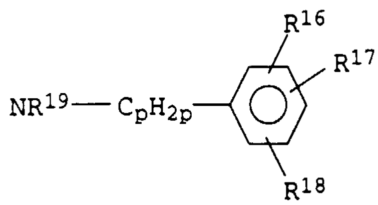 ,

worin p = O oder 1 bis 22 sein kann und $R^{16}$, $R^{17}$ und $R^{18}$ unabhängig voneinander für H, Alkyl mit 1 bis 22 Kohlenstoffatomen, O-$C_1$- bis $C_{22}$-Alkyl,
S-$C_1$- bis $C_{22}$-Alkyl, ;

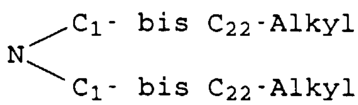 ;

$$CH_2-(O-CH_2-CH_2)_{1-6}-R^{14}\;;$$

$$CH_2-(O-CH_2-CH(CH_3))_{1-6}-R^{14}\;;$$

$$CH_2-CH_2-(O-CH_2-CH_2)_{1-6}-R^{14}\;;$$

$$CH_2-CH_2-(O-CH_2-CH(CH_3))_{1-6}-R^{14}\;;$$

Halogen, $NO_2$, CN,

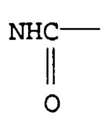 ;

$R^{19}$ für Alkyl mit 1 bis 20 Kohlenstoffatomen,

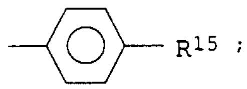

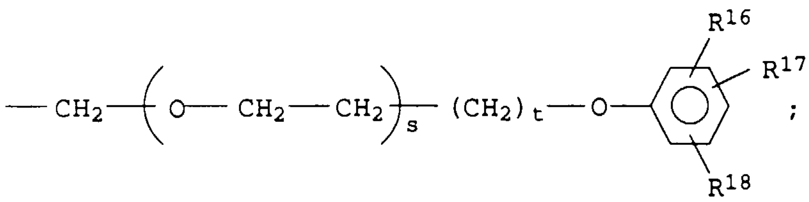

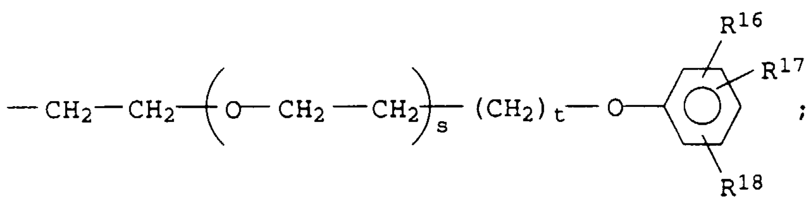

mit s = 0,1 oder 2
und t = 0 oder 1 stehen,
ein fünf- oder sechsgliedriger stickstoff-, sauerstoff- oder schwefelhaltiger heteroaromatischer Rest, der gegebenenfalls durch Alkyl mit 1 bis 22 Kohlenstoffatomen,
$O-C_1$- bis $C_{22}$-Alkyl, $S-C_1$- bis $C_{22}$-Alkyl, $NH-C_1$ - bis $C_{22}$-Alkyl
oder

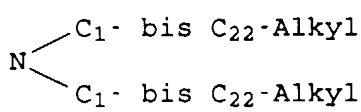

substituiert sein kann,
$-C{\equiv}CH$ $-CH_2-C{\equiv}CH$, $-CH_2-C{\equiv}C-CH_3$, $-CH_2-C{\equiv}C-C_2H_5$, $-CH_2-CH_2-C{\equiv}CH$,

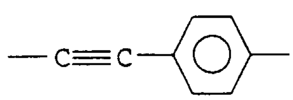

$C_1$- bis $C_{22}$-Alkyl ;

A für $-(CH_2)-_{4\ bis\ 8}$, $-CH_2-O-CH_2-CH_2-OCH_2-$,
$-CH_2-O-CH_2-CH(CH_3)-O-CH_2-$,
$-CH_2-O-CH_2-CH_2-CH_2-O-CH_2-$,
$-CH_2-O-(CH_2)_4-O-CH_2-$,
$-O\{CH_2-CH_2-O\}_{1\ oder\ 2}$,
$-O\{CH_2-CH(CH_3)-O\}_{1\ oder\ 2}$ oder

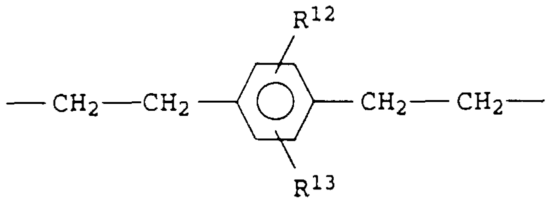 ,

worin

$R^{12}$ und $R^{13}$ unabhängig voneinander die oben angegebene Bedeutung haben sowie zusätzlich Halogen, CN, $NO_2$, $-N=N-R^{20}$ oder

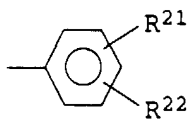

bedeuten können, wobei

$R^{20}$ für eine Phenylgruppe oder eine mit $NO_2$, CN, Halogen, $C_1$- bis $C_4$-Alkyl, $C_1$, bis $C_4$-Alkoxy, $C_1$- bis $C_4$-alkylthio oder $C_1$- bis $C_4$-alkylamino substituierte Phenylgruppe,

$R^{21}$ und $R^{22}$ unabhängig voneinander für $C_1$- bis $C_4$-Alkyl, $C_1$- bis $C_4$-Alkoxy, CN, $NO_2$ oder Halogen stehen,

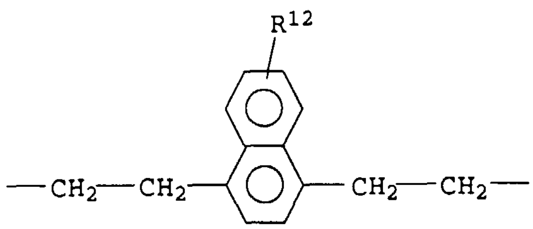 ,

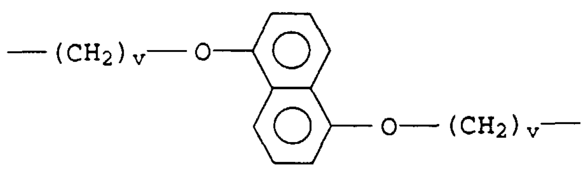 ,

mit v = 1, 2 oder 3

oder

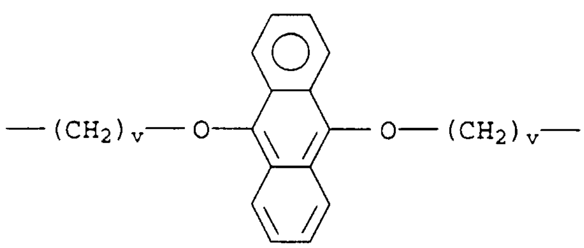 ,

stehen.

**3.** Verbindungen nach Anspruch 1, dadurch gekennzeichnet, daß als axial-chirale Gruppen solche der Formeln

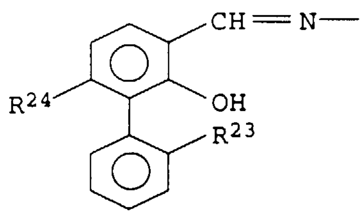

oder

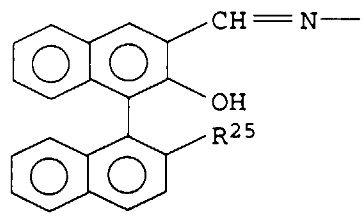

worin

$R^{23}$ und $R^{24}$ unabhängig voneinander für $C_1$- bis $C_{22}$-Alkyl, $C_1$- bis $C_6$-Alkyloxy, Phenyl, mit Halogen, CN, $NO_2$, $C_1$- bis $C_4$-Alkyl, $C_1$- bis $C_4$-Alkoxy substituiertes Phenyl, oder

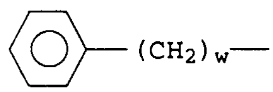

mit w = 1 bis 4 stehen,

mit der Maßgabe, daß mindestens einer der Reste $R^{23}$ und $R^{24}$ eine tert.-Butylgruppe oder ein anderer die freie 360° Drehbarkeit der Phenylreste verhindernder Rest ist und $R^{25}$ ein tert. Butylrest, ein Phenylrest oder ein mit Halogen, CN, $NO_2$, $C_1$- bis $C_4$-Alkyl oder $C_1$ bis $C_4$-Alkoxy substituierter Phenylrest ist,

mit dendritischen stickstoffhaltigen organischen Verbindungen der allgemeinen Formel (I) verknüpft sind, worin =N- jeweils der Stickstoff der aus der primären Aminogruppe erhaltenen Schiff'schen Base ist.

**4.** Verbindungen nach Anspruch 2, dadurch gekennzeichnet, daß sie als Metallkomplexe von Übergangsmetallen vorliegen.

**5.** Verbindungen nach Anspruch 3, dadurch gekennzeichnet, daß sie als Metallkomplexe von Übergangsmetallen vorliegen.

**6.** Verfahren zur Herstellung dendritischer stickstoffhaltiger organischer Verbindungen nach Anspruch 2, die mindestens 4 planar-chirale Gruppen enthalten, dadurch gekennzeichnet, daß Verbindungen der allgemeinen Formel (I) gemäß Anspruch 1 mit Hydroxyaldehyden der Formel

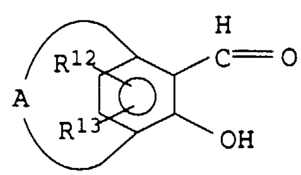

miteinander umgesetzt werden.

**7.** Verfahren zur Herstellung dendritischer stickstoffhaltiger organischer Verbindungen nach Anspruch 3, die mindestens 4 axial-chirale Gruppen enthalten, dadurch gekennzeichnet, daß Verbindungen der allgemeinen Formel (I) gemäß Anspruch 1 mit Hydroxyaldehyden der Formeln, worin $R^{23}$ bis $R^{25}$ die in Anspruch 3 angegebene Bedeutung haben,

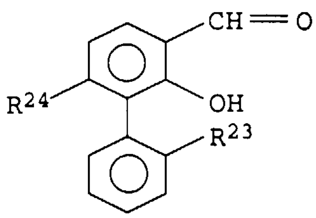

oder

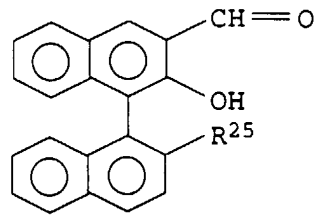

miteinander umgesetzt werden.

**8.** Verfahren zur Herstellung von Metallkomplexen nach Anspruch 4 oder 5, dadurch gekennzeichnet, daß die dendritischen planar-chirale bzw. axial-chirale Gruppen enthaltenden Verbindungen zusammen mit Übergangsmetallsalzen in einem geeigneten Lösungsmittel bei einer Temperatur zwischen Raumtemperatur und Siedetemperatur des Lösungsmittels gelöst oder dispergiert, das Lösungsmittel anschließend entfernt, die erhaltenen Metallkomplexe durch Behandeln mit Wasser von überschüssigem Metallsalz befreit wird.

**9.** Verfahren zur Herstellung von Metallkomplexen nach Anspruch 4 oder 5, dadurch gekennzeichnet, daß die Übergangsmetallsalze der Reaktionsmischung zur Herstellung der Schiff'schen Basen zugefügt werden.

**10.** Verwendung von dendritischen stickstoffhaltigen organischen Verbindungen nach einem der Ansprüche 1 bis 3 als Katalysatoren für die asymmetrische homogene Katalyse.

**11.** Verwendung von Metallkomplexen nach Anspruch 4 oder 5 als Katalysatoren für die asymmetrische homogene Katalyse.

**Claims**

**1.** A dendritic nitrogen-containing organic compound which contains at least 4 planar-chiral or axial-chiral groups, where these planar-chiral or axial-chiral groups are linked as Schiff's bases to the primary amino groups of compounds of the formula (I)

$$(R^1R^1)N\text{-}X\text{-}N(R^1R^1) \qquad (I)$$

where

$R^1$ is $(R^2R^2)N\text{-}(CH_2)_2\text{-}$ or $(R^2R^2)N\text{-}(CH_2)_3\text{-}$,

$R^2$ is hydrogen or $(R^3R^3)N\text{-}(CH_2)_2\text{-}$ or $(R^3R^3)N\text{-}(CH_2)_3\text{-}$,

$R^3$ is hydrogen or $(R^4R^4)N\text{-}(CH_2)_2\text{-}$ or $(R^4R^4)N\text{-}(CH_2)_3\text{-}$,

$R^4$ is hydrogen or $(R^5R^5)\text{-}N\text{-}(CH_2)_2\text{-}$ or $(R^5R^5)N\text{-}(CH_2)_3\text{-}$,

$R^5$ is hydrogen or $(R^6R^6)N\text{-}(CH_2)_2\text{-}$ or $(R^6R^6)N\text{-}(CH_2)_3\text{-}$ and

$R^6$ is hydrogen

x is

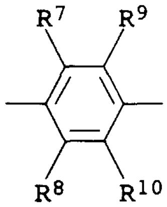 , 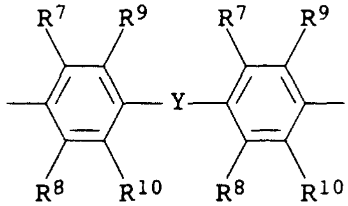 , 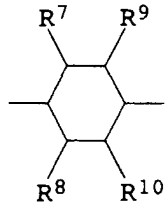 ,

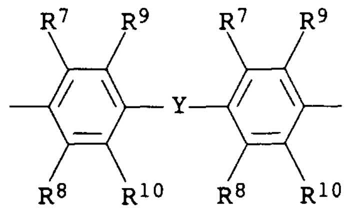 ,

$-(CH_2)_n-$, $-(CH_2)_3-NR^{11}-(CH_2)_3-$, $(CH_2)_2-NR^{11}-(CH_2)_2-$, $C_2$- bis $C_{20}$-Alkylen
$-(CH_2)_1-$ $[O-(CH_2)_k]_m-O-(CH_2)_1)-$

where Y is $CR^7R^9$, oxygen, C=O or $SO_2$, n is from 2 to 20, l and k are from 2 to 6, and m is from 1 to 40,

| | |
|---|---|
| $R^7$, $R^8$, $R^9$ and $R^{10}$ | are hydrogen or $C_1$- to $C_6$-alkyl, |
| $R^{11}$ | is $C_1$- to $C_{20}$-alkyl, $C_2$- to $C_{20}$-dialkylamino-$C_2$- to $C_{10}$-alkyl, $C_1$- to $C_{10}$-alkoxy-$C_2$- to $C_{10}$-alkyl, $C_2$- to $C_{20}$-hydroxyalkyl, $C_3$- to $C_{12}$-cycloalkyl, $C_4$- to $C_{20}$-cycloalkylalkyl, $C_2$- to $C_{20}$-alkenyl, $C_4$- to $C_{30}$-dialkylaminoalkenyl, $C_3$- to $C_{30}$-alkoxyalkenyl, $C_3$- to $C_{20}$-hydroxyalkenyl, $C_5$- to $C_{20}$-cycloalkylalkenyl, aryl or $C_7$- to $C_{20}$-aralkyl which is unsubstituted or monosubstituted to pentasubstituted by $C_1$- to $C_8$-alkyl, $C_2$- to $C_8$-dialkylamino, $C_1$- to $C_8$-alkoxy, hydroxyl, $C_3$-to $C_8$-cycloalkyl or $C_4$- to $C_{12}$-cycloalkylalkyl, or together are an alkylene chain which may be interrupted by nitrogen or oxygen, such as ethylene oxide, propylene oxide, butylene oxide or $-CH_2-CH(CH_3)-O-$ or polyisobutylene having 1 to 100 isobutylene units. |

**2.** A compound as claimed in claim 1, wherein the planar-chiral groups linked to dendritic nitrogen-containing organic compounds of the formula (I) are those of the formula

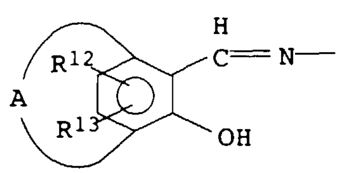

where =N- is in each case the nitrogen of the Schiff's base obtained from the primary amino group,

$R^{12}$ and $R^{13}$, independently of one another, can have the following meanings:
H, alkyl having 1 to 22 carbon atoms,
$O-C_1$- to $C_{22}$-alkyl,
$S-C_1$- to $C_{22}$-alkyl,

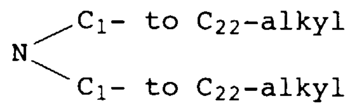
,
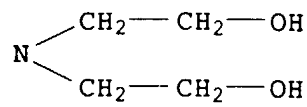

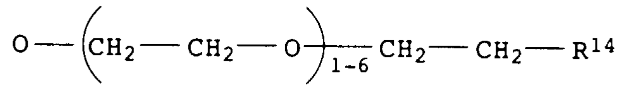

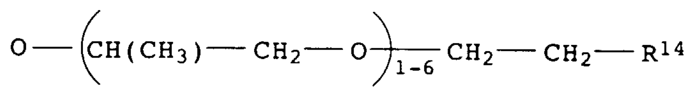

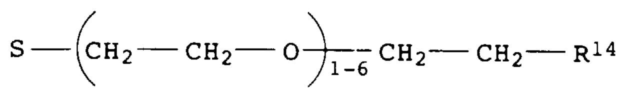

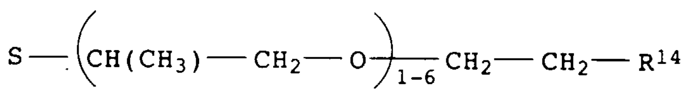
,

where

$R^{14}$ is H, OH, $OCH_3$, $OC_2H_5$,

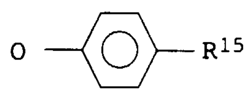

or

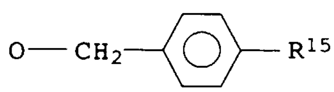
,

and

$R^{15}$ is H, alkyl having 1 to 4 carbon atoms, O-$C_1$- to $C_4$-alkyl, halogen, CN or $NO_2$,

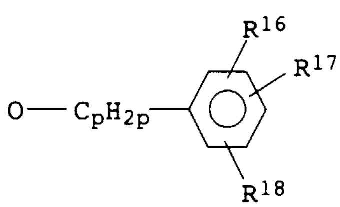
,

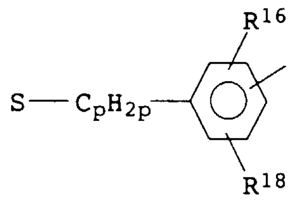 ,

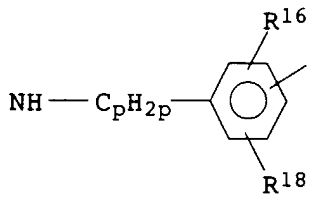 ,

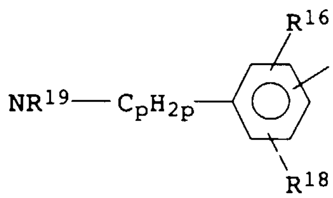 ,

in which p can be 0 or from 1 to 22, and $R^{16}$, $R^{17}$ and $R^{18}$, independently of one another, are H, alkyl having 1 to 22 carbon atoms, O-$C_1$- to $C_{22}$-alkyl,

S-$C_1$- to $C_{22}$-alkyl, 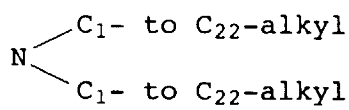 ;

$$CH_2-(O-CH_2-CH_2)_{1-6}-R^{14};$$

$$CH_2-(O-CH_2-CH(CH_3))_{1-6}-R^{14};$$

$$CH_2-CH_2-(O-CH_2-CH_2)_{1-6}-R^{14};$$

$$CH_2-CH_2-(O-CH_2-CH(CH_3))_{1-6}R^{14};$$

Halogen, $NO_2$, CN,

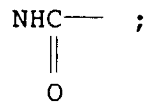

$R^{19}$ is alkyl having 1 to 20 carbon atoms,

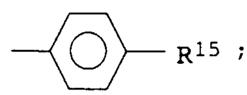

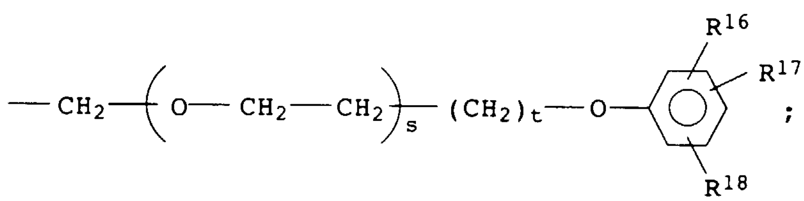

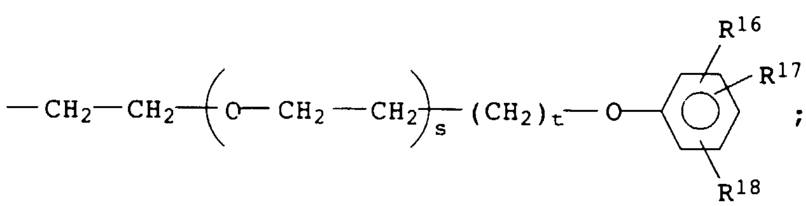

where s = 0, 1 or 2
and t = 0 or 1,
a five- or six-membered nitrogen-, oxygen- or sulfur-containing heteroaromatic radical, which is unsubstituted or substituted by alkyl having 1 to 22 carbon atoms,
O-$C_1$- to $C_{22}$-alkyl, S-$C_1$- to $C_{22}$-alkyl, NH-$C_1$- to $C_{22}$-alkyl
or

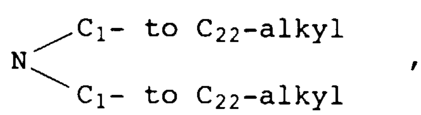

$-C\equiv CH$, $-CH_2-C\equiv CH$, $-CH_2-C\equiv C-CH_3$, $-CH_2-C\equiv C-C_2H_5$, $-CH_2-CH_2-C\equiv CH$,

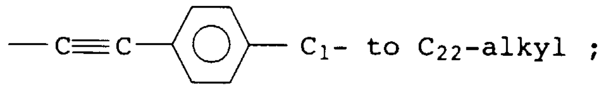

and A is $-(CH_2)-_{4\ to\ 8}$, $-CH_2-O-CH_2-CH_2-OCH_2-$,
$-CH_2-O-CH_2-CH(CH_3)-O-CH_2-$,
$-CH_2-O-CH_2-CH_2-CH_2-O-CH_2-$,
$-CH_2-O-(CH_2)_4-O-CH_2-$,
$-O\{CH_2-CH_2-O\}_{1\ or\ 2}$,
$-O\{CH_2-CH(CH_3)-O\}_{1\ or\ 2}$ or

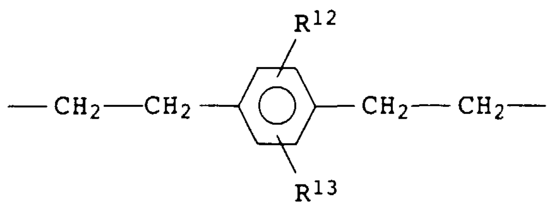,

where

$R^{12}$ and $R^{13}$, independently of one another, are as defined above or are halogen, CN, $NO_2$, $-N=N-R^{20}$ or

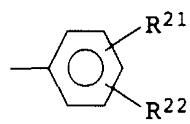

where

$R^{20}$ is phenyl which is unsubstituted or substituted by $NO_2$, CN, halogen, $C_1$- to $C_4$-alkyl, $C_1$- to $C_4$-alkoxy, $C_1$- to $C_4$-alkylthio or $C_1$- to $C_4$-alkylamino,

$R^{21}$ and $R^{22}$, independently of one another, are $C_1$- to $C_4$-alkyl, $C_1$- to $C_4$-alkoxy, CN, $NO_2$ or halogen,

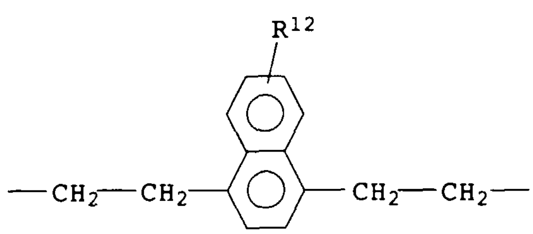,

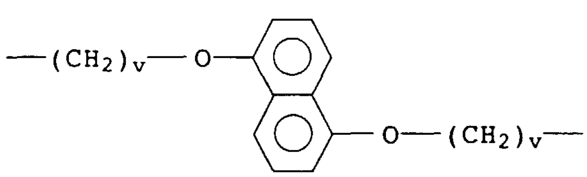,

where v = 1, 2 or 3

or

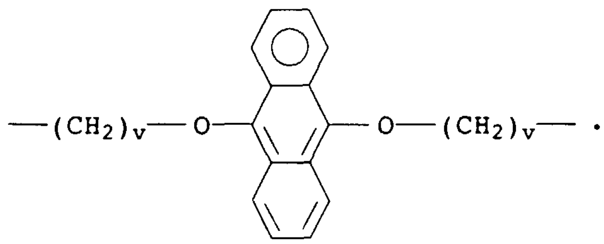

3. A compound as claimed in claim 1, wherein the axial-chiral groups linked to dendritic nitrogen-containing organic compounds of the formula (I) are those of the formula

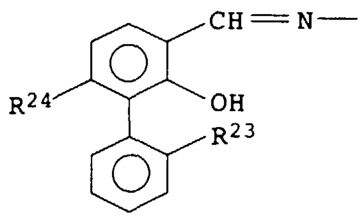

or

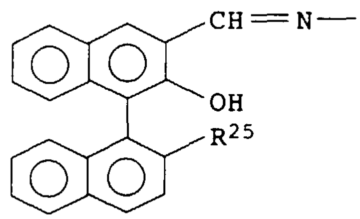

where
=N- is in each case the nitrogen of the Schiff's base obtained from the primary amino group,

$R^{23}$ and $R^{24}$, independently of one another, are $C_1$- to $C_{22}$-alkyl, $C_1$- to $C_6$-alkyloxy or phenyl which is unsubstituted or substituted by halogen, CN, $NO_2$, $C_1$- to $C_4$-alkyl or $C_1$- to $C_4$-alkoxy, or

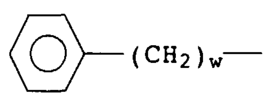

where w = 1 to 4,
with the proviso that at least one of $R^{23}$ and $R^{24}$ is tert-butyl or a radical which prevents the free 360° rotatability of the phenyl radicals, and $R^{25}$ is tert-butyl or phenyl which is unsubstituted or substituted by halogen, CN, $NO_2$, $C_1$- to $C_4$-alkyl or $C_1$- to $C_4$-alkoxy.

4. A compound as claimed in claim 2, in the form of a metal complex with a transition metal.

5. A compound as claimed in claim 3, in the form of a metal complex with a transition metal.

6. A process for the preparation of a dendritic, nitrogen-containing organic compound as claimed in claim 2 containing at least 4 planar-chiral groups, which comprises reacting a compound of the formula (I) as claimed in claim 1 with a hydroxyaldehyde of the formula

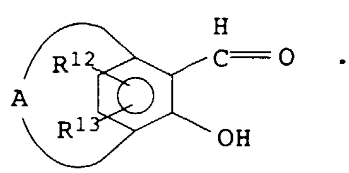

7. A process for the preparation of a dendritic, nitrogen-containing organic compound as claimed in claim 3 containing

at least 4 axial-chiral groups, which comprises reacting a compound of the formula (I) as claimed in claim 1 with a hydroxyaldehyde of the formula

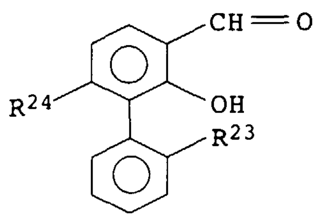

or

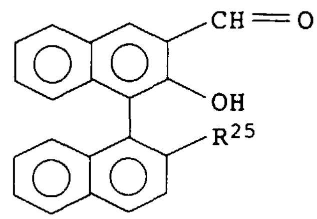

where $R^{23}$ to $R^{25}$ are as defined in claim 3.

**8.** A process for the preparation of a metal complex as claimed in claim 4 or 5, which comprises dissolving or dispersing a dendritic compound containing planar-chiral or axial-chiral groups together with a transition-metal salt in a suitable solvent at a temperature between room temperature and the boiling point of the solvent, subsequently removing the solvent, and freeing the resultant metal complex from excess metal salt by treatment with water.

**9.** A process for the preparation of a metal complex as claimed in claim 4 or 5, which comprises adding a transition-metal salt to the reaction mixture for the preparation of the Schiff's base.

**10.** The use of a dendritic, nitrogen-containing organic compound as claimed in any of claims 1 to 3 as a catalyst for asymmetrical homogeneous catalysis.

**11.** The use of a metal complex as claimed in claim 4 or 5 as a catalyst for asymmetrical homogeneous catalysis.

## Revendications

**1.** Composés organiques azotés- dendritiques contenant au moins quatre groupes chiraux-planaires ou chiraux-planaires ces groupes chiro-planaires ou chiraux-axiaux étant reliés à l'état de bases de Schiff avec les groupes amino primaires de composés de formule générale I

$$(R^1R^1)N\text{-}X\text{-}N(R^1R^1) \qquad (I)$$

dans laquelle

| | |
|---|---|
| $R^1$ | représente $(R^2R^2)N\text{-}(CH_2)_2\text{-}$ ou $(R^2R^2)N\text{-}(CH_2)_3\text{-}$, |
| $R^2$ | représente l'hydrogène ou $(R^3R^3)N\text{-}(CH_2)_2\text{-}$ ou $(R^3R^3)N\text{-}(CH_2)_3\text{-}$, |
| $R^3$ | représente l'hydrogène ou $(R^4R^4)N\text{-}(CH_2)_2\text{-}$ ou $(R^4R^4)N\text{-}(CH_2)_3\text{-}$, |
| $R^4$ | représente l'hydrogène ou $(R^5R^5)\text{-}N\text{-}(CH_2)_2\text{-}$ ou $(R^5R^5)N\text{-}(CH_2)_3\text{-}$, |
| $R^5$ | représente l'hydrogène ou $(R^6R^6)N\text{-}(CH_2)_2\text{-}$ ou $(R^6R^6)N\text{-}(CH_2)_3\text{-}$ et |
| $R^6$ | représente l'hydrogène, |
| X | représente un groupe |

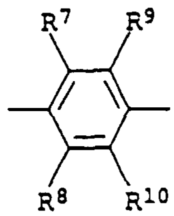 , 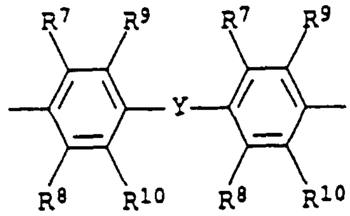 , 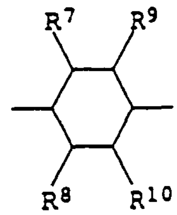 ,

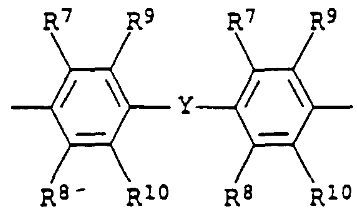 ,

$-(CH_2)_n-$, $-(CH_2)_3-NR^{11}-(CH_2)_3-$, $(CH_2)_2-NR^{11}-(CH_2)_2-$, $C_2$- bis $C_{20}$-Alkylène en $C_2$-$C_{20}$ -$(CH_2)_1$- [O- $(CH_2)_k]_m$-O-$(CH_2)_1)$-

et Y représente $CR^7R^9$, l'oxygène, C=O, $SO_2$, n est un nombre allant de 2 à 20, 1 et k sont des nombres allant de 2 à 6 et m est un nombre allant de 1 à 40,

$R^7$, $R^8$, $R^9$, $R^{10}$ représentent chacun l'hydrogène ou un groupe alkyle en C1-C6,

$R^{11}$ représente un groupe alkyle en C1-C20, dialkylamino en C2-C20, alkyle en C2-C10, (alcoxy en C1-C10)alkyle en C2-C10, hydroxyalkyle en C2-C20, cycloalkyle en C3-C12, cycloalkylalkyle en C4-C20, alcényle en C2-C20, dialkylaminoalcényle en C4-C30, alcoxyalcényle en C3-C30, hydroxyalcényle en C3-C20, cycloalkylalcényle en C5-C20, aryle portant éventuellement un à cinq substituants alkyle en C1-C8, dialkylamino en C2-C8, alcoxy en C1-C8, hydroxy, cycloalkyle en C3-C8, cycloalkylalkyle en C4-C12, ou un groupe aralkyle en C7-C20, ou bien, ensemble, une chaîne alkylène éventuellement interrompue par l'azote ou l'oxygène, par exemple une chaîne oxyde d'éthylène, oxyde de propylène, oxyde de butylène ou $-CH_2-CH(CH_3)-O-$ ou polyisobutylène de un à cent motifs isobutylène.

**2.** Composés selon la revendication 1, caractérisés par le fait que les groupes chiraux planaires reliés avec les composés organiques azotés dendritiques de formule générale I sont des groupes de formule

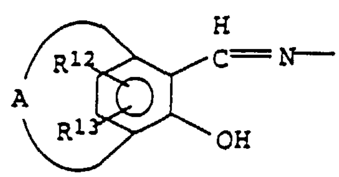

dans laquelle =N- représente dans chaque cas l'azote de la base de Schiff obtenue à partir du groupe amino primaire,

$R^{12}$ et $R^{13}$ peuvent avoir, indépendamment l'un de l'autre, les significations suivantes :

H, un groupe alkyle en C1-C22,
O-alkyle en C1-C22,
S-alkyle en C1-C22,

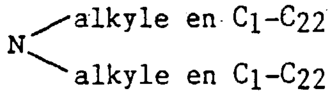 , 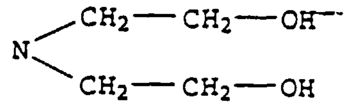

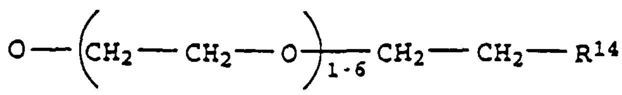

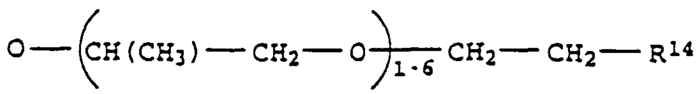

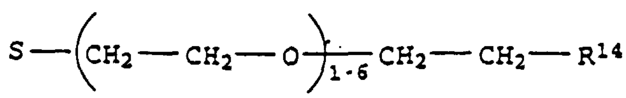

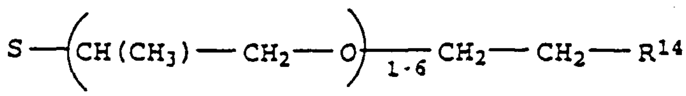 ,

$R^{14}$ représentant H, OH, $OCH_3$, $OC_2H_5$,

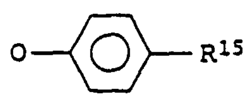

ou

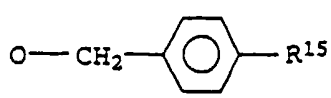

et

$R^{15}$ représente H, un groupe alkyle en C1-C4, O-alkyle en C1-C4, un halogène, un groupe CN ou $NO_2$,

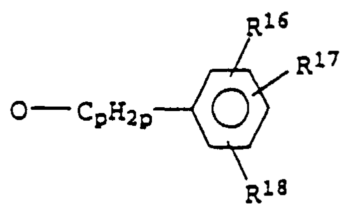 ,

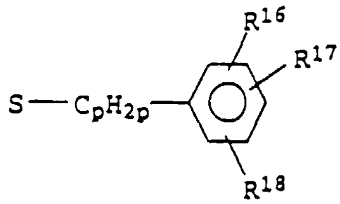
,

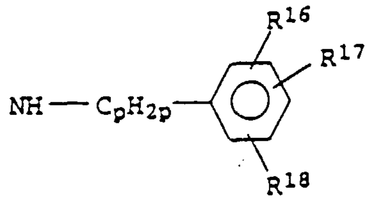
,

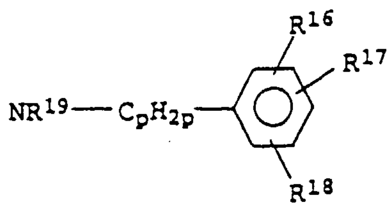
,

dans lesquels p est égal à O ou peut prendre une valeur de 1 à 22 et $R^{16}$, $R^{17}$ et $R^{18}$ représentent chacun, indépendamment les uns des autres, H, un groupe alkyle en C1-C22, O-alkyle en C1-C22, S-alkyle en C1-C22 ;

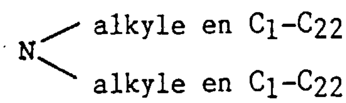
;

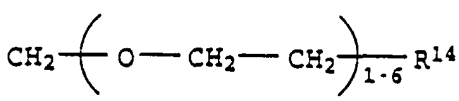
;

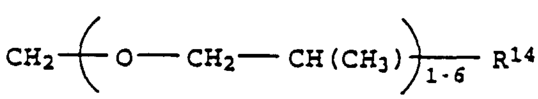
;

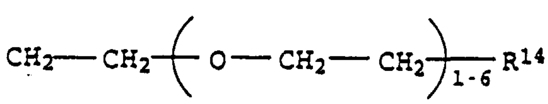
;

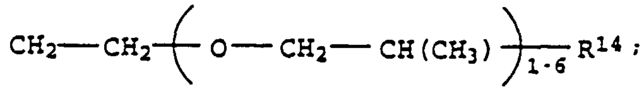

un halogène, un groupe $NO_2$, CN,

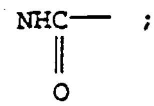

R[19] représente un groupe alkyle en C1-C20,

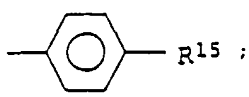

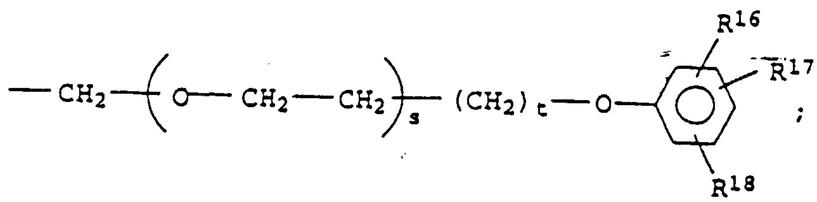

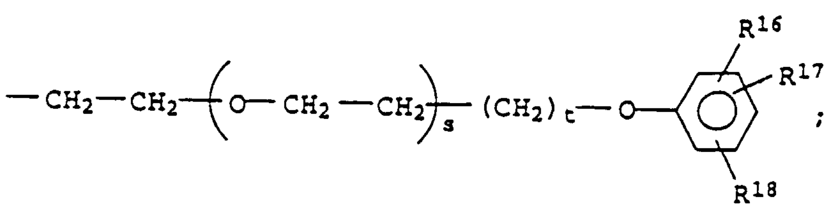

dans lesquels s = 0, 1 ou 2 et
t = 0 ou 1,
un radical hétéroaromatique azoté, oxygéné ou sulfuré de cinq ou six chaînons qui peut le cas échéant être substitué par des groupes alkyle en C1-C22, O-alkyle en C1-C22, S-alkyle en C1-C22, NH-alkyle en C1-C22,
ou bien par

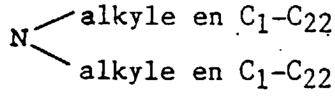

un groupe -C≡-CH , $CH_2$-C≡CH, -$CH_2$-C≡-$CH_3$, -$CH_2$-C≡C-$C_2H_5$, -$CH_2$-$CH_2$-C≡CH,

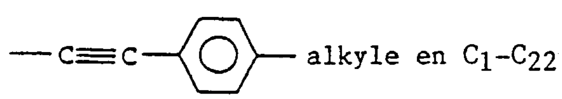

A représente $-(CH_2)-_{4\ à\ 8}$, $-CH_2-O-CH_2-CH_2-OCH_2-$,
$-CH_2-O-CH_2-CH(CH_3)-O-CH_2-$,
$-CH_2-O-CH_2-CH_2-CH_2-O-CH_2-$,
$-CH_2-O-(CH_2)_4-O-CH_2-$,
$-O\{CH_2-CH_2-O\}_{1\ ou\ 2}$,
$-O\{CH_2-CH(CH_3)-O\}_{1\ ou\ 2}$ ou bien

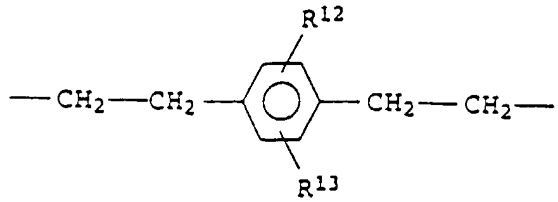 ,

dans lesquels

$R^{12}$ et $R^{13}$ peuvent avoir, indépendamment l'un de l'autre, les significations indiquées ci-dessus ou bien représenter en outre un halogène, un groupe CN, $NO_2$, $-N=N-R^{20}$ ou

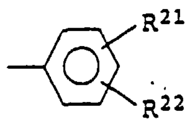

dans lesquels

$R^{20}$ représente un groupe phényle ou un groupe phényle portant des substituants $NO_2$, CN, halogéno, alkyle en C1-C4, alcoxy en C1-C4, alkylthio en C1-C4 ou alkylamino en C1-C4,

$R^{21}$ et $R^{22}$ représentent chacun, indépendamment l'un de l'autre, un groupe alkyle en C1-C4, alcoxy en C1-C4, CN, $NO_2$ ou un halogène, un groupe

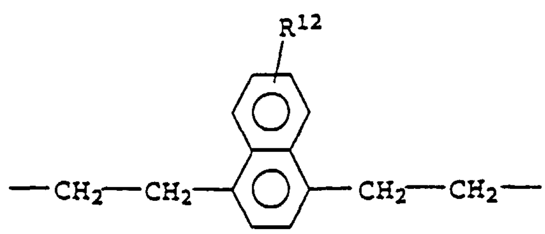 ,

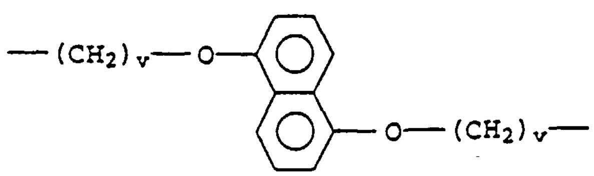 ,

avec v = 1, 2 ou 3
ou

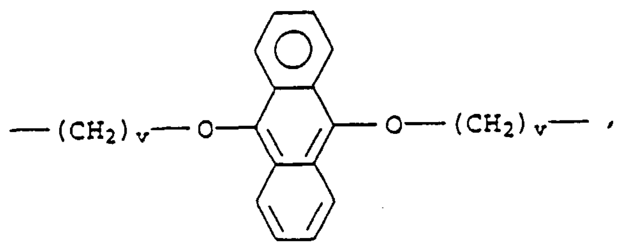

**3.** Composés selon la revendication 1, caractérisé par le fait que les groupes chiraux-axiaux reliés avec les composés organiques azotés dendritiques de formule générale I sont des groupes de formule

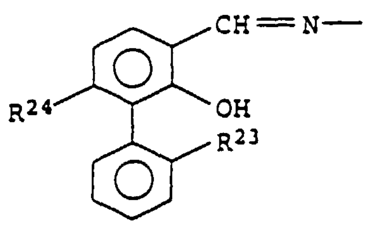

ou

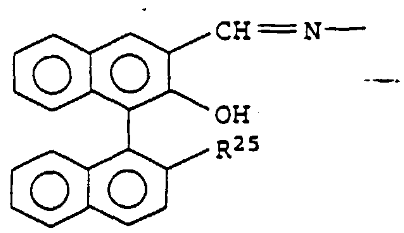

dans lesquelles

$R^{23}$ et $R^{24}$ représentent chacun, indépendament l'un de l'autre, un groupe alkyle en C1-C22, alcoxy en C1-C6, phényle, phényle portant des substituants halogéno, CN, $NO_2$, alkyle en C1-C4, alcoxy en C1-C4, ou bien

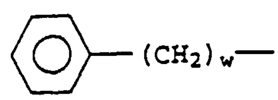

avec w = 1 à 4
sous réserve que l'un au moins des groupes $R^{23}$ et $R^{24}$ est un groupe tert-butyle ou un autre groupe empêchant la rotation libre de 360° des groupes phényle, et $R^{25}$ est un groupe tert-butyle, un groupe phényle ou un groupe phényle portant des substituants halogéno, CN, $NO_2$, alkyle en C1-C4 ou alcoxy en C1-C4,
=N- représentant dans chaque cas l'azote de la base de Schiff obtenue à partir du groupe amino primaire.

**4.** Composés selon la revendication 2, caractérisés par le fait qu'ils sont à l'état de complexes métalliques de métaux de transition.

**5.** Composés selon la revendication 3, caractérisés par le fait qu'ils sont à l'état de complexes métalliques de métaux de transition.

**6.** Procédé de préparation de composés organiques azotés dendritiques selon la revendication 2 contenant au moins quatre groupes chiraux-planaires, caractérisé par le fait que l'on fait réagir entre eux des composés de formule générale I de la revendication 1 et des hydroxyaldéhydes de formule

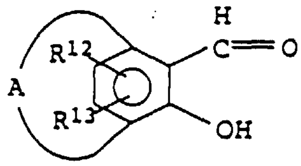

**7.** Procédé de préparation des composés organiques azotés dendritiques selon la revendication 3, contenant au moins quatre groupes chiraux-axiaux, caractérisé par le fait que l'on fait réagir entre eux des composés de formule générale I selon la revendication 1 et des hydroxyaldéhydes de formules

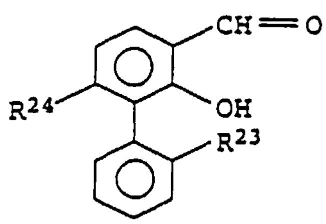

ou

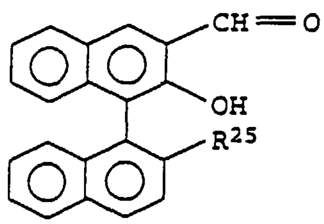

dans lesquelles $R^{23}$ à $R^{25}$ ont les significations indiquées dans la revendication 3.

**8.** Procédé de préparation des complexes métalliques selon la revendication 4 ou 5, caractérisé par le fait que l'on dissout ou l'on disperse les composés dendritiques contenant des groupes chiraux-planaires ou chiraux-axiaux avec des sels de métaux de transition dans un solvant approprié à une température comprise entre la température ambiante et la température d'ébullition du solvant, on élimine ensuite le solvant et on libère les complexes métalliques formés de l'excès de sel métallique par traitement à l'aide d'eau.

**9.** Procédé de préparation des complexes métalliques selon la revendication 4 ou 5, caractérisé par le fait que les sels de métaux de transition sont ajoutés au mélange de réaction servant à la préparation des bases de Schiff.

**10.** Utilisation des composés organiques azotés dendritiques selon l'une des revendications 1 à 3 en tant que catalyseurs dans des catalyses homogènes asymétriques.

**11.** Utilisation des complexes métalliques selon la revendication 4 ou 5 en tant que catalyseurs pour des catalyses homogènes asymétriques.

FIG.1

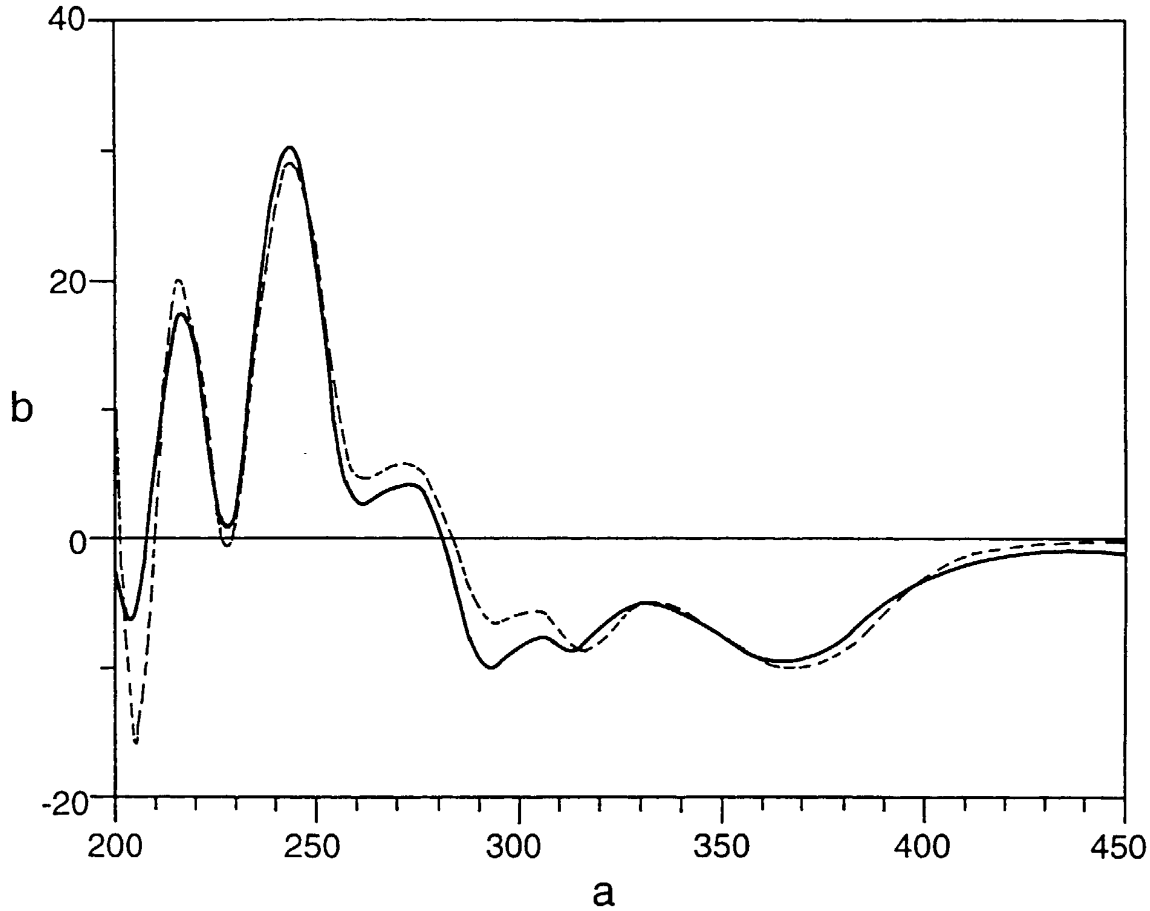

FIG.2